(19) Europäisches Patentamt
European Patent Office
Office européen des brevets

(11) EP 1 686 183 A1

(12) **EUROPEAN PATENT APPLICATION**
published in accordance with Art. 158(3) EPC

(43) Date of publication:
**02.08.2006 Bulletin 2006/31**

(21) Application number: **04818202.6**

(22) Date of filing: **05.11.2004**

(51) Int Cl.:
***C12P 7/46*** *(2006.01)*

(86) International application number:
**PCT/JP2004/016437**

(87) International publication number:
**WO 2005/045049 (19.05.2005 Gazette 2005/20)**

(84) Designated Contracting States:
**DE FR**

(30) Priority: **07.11.2003 JP 2003378732**
**19.03.2004 JP 2004079488**

(71) Applicants:
- **MITSUBISHI CHEMICAL CORPORATION**
**Tokyo 108-0014 (JP)**
- **Ajinomoto Co., Inc.**
**Tokyo 104-8315 (JP)**

(72) Inventors:
- **Isotani, Atsushi**
**Mitsubishi Chemical Group Science**
**Yokkaichi-shi, Mie;5108530 (JP)**
- **Ikeda, Hideo**
**c/o Mitsubishi Chemical Group Science**
**Yokkaichi-shi, Mie;5108530 (JP)**
- **Yamagishi, K.**
**Mitsubishi Chemical Gr. Science and**
**Yokohama-shi,Kanagawa, 2278502 (JP)**
- **Aoyama, R.**
**Mitsubishi Chemical Group Science and**
**Yokohama-shi, Kanagawa, 2278502 (JP)**

(74) Representative: **HOFFMANN EITLE**
**Patent- und Rechtsanwälte**
**Arabellastrasse 4**
**81925 München (DE)**

(54) **METHOD FOR PRODUCING ORGANIC ACID AMMONIUM SOLUTION**

(57) Organic acid ammonium solution is produced by the steps of obtaining a fermentation broth containing organic acid magnesium by using an organic acid-producing microorganism in the presence of a magnesium compound, producing organic acid ammonium and a magnesium compound by subjecting the organic acid magnesium contained in the fermentation broth to salt-exchange using an ammonia compound, and separating the produced magnesium compound to obtain organic acid ammonium solution.

EP 1 686 183 A1

## Description

Technical Field

The present invention relates to a method for producing organic acid ammonium solution such as ammonium succinate solution. More specifically, the present invention relates to a method for producing organic acid ammonium solution which is suitable for producing an organic acid by microbial conversion from a raw material of biologic origin such as glucose, dextrose, and cellulose.

Background Art

Organic acids include fumaric acid, maleic acid, malic acid, and succinic acid. Of those, succinic acid and its derivative are widely used as a raw material for polymers such as biodegradable polyester and polyamide, or a raw material for foods, pharmaceuticals, cosmetics, or the like.

An organic acid such as succinic acid has been industrially produced so far, by hydrogenation of maleic acid, which is a raw material derived from petroleum. Recently, there has been studied the production of an organic acid from a raw material derived from a plant by methods utilizing a fermentation operation.

In the production of an organic acid by fermentation, pH of a medium decreases together with generation of the organic acid. However, microorganisms used in the fermentation generally exhibit insufficient activity under low pH conditions and a fermentation solution must be neutralized. Examples of a neutralizer generally used include sodium hydroxide, sodium bicarbonate, sodium carbonate, ammonia, ammonium carbonate, ammonium bicarbonate, urea, calcium hydroxide, calcium carbonate, magnesium hydroxide, and magnesium carbonate. However, when such a neutralizer is used, an organic acid to be obtained from a fermentation tank is in a form of a salt with alkali, and thus a distillation operation as a general separation/purification technique cannot be used.

Under such circumstances, methods of separating/purifying an organic acid from an organic acid salt formed by fermentation are proposed such as a method using electrodialysis (Patent Document 1); a method using an ion-exchange resin (Patent Document 2); a method of decomposing calcium succinate, which has been produced by fermentation while neutralizing with calcium hydroxide, by using sulfuric acid (Patent Document 3); a method of performing reactive crystallization by a salt-exchange reaction with sulfuric acid (Patent Document 4 or 5); and a reactive extraction method (Patent Document 6).

In fermentation, a particularly preferable neutralizer is restricted depending on properties of a microorganism. Meanwhile, each of the above-mentioned purification methods cannot necessarily be employed for any neutralizer, and employed for a restricted neutralizer. Thus, selection of the neutralizer in the fermentation and restriction of the neutralizer in the purification must conform absolutely with each other.

In the method using electrodialysis, the neutralizer must be a monovalent cation. A divalent cation precipitates as gypsum in an electrodialysis membrane, and significantly degrades performance of the membrane. Thus, ammonia, sodium, and potassium are preferable as a neutralizer.

In the method using an ion-exchange resin, by-product salt is formed, and a neutralizer that is supplied stably and has a low market price should be selected. Thus, ammonia and sodium are preferable as a neutralizer, and potassium and calcium, and magnesium follows them in this order. However, formation of by-product salt requires a treatment cost, and the method using an ion-exchange resin is not a very preferable method.

In the method using reactive extraction with amine, the neutralizer remains as a carbonate salt in an aqueous phase. Thus, the carbonate salt as a neutralizer precipitates if the solubility into water is too low, and an operation in high-pressure extraction tower cannot be performed. Thus, ammonia, sodium, and potassium are preferable as a neutralizer.

In the method using acid precipitation with sulfuric acid, a sulfate salt is formed as by-product (gypsum method: Patent Document 3). Thus, as in the method using an ion-exchange resin, a neutralizer that is stably supplied and has a low market price should be selected. Furthermore, formation of by-product salt requires a treatment cost, and the method using acid precipitation is not a very preferable method.

As a solution to this problem, Patent Documents 4 and 5 propose a method comprising heat-decomposing ammonium sulfate at 300°C or higher, recycling sulfuric acid as monoammonium sulfate, and using ammonia as a neutralizer. These documents propose a method of converting sodium salt into ammonium salt when sodium is used as a neutralizer in fermentation step.

That is, ammonium sulfate has an advantage of preventing formation of by-product salt when ammonia is used as a neutralizer. The same results can be attained by using monoammonium sulfate for recovery of resin even in the method using an ion-exchange resin.

As described above, ammonia neutralization can be applied to most of diverse purification methods. When the method described in Patent Documents 4 and 5 is applied, ammonia is a neutralizer having an advantage of preventing formation of by-product salt.
Sodium can be converted into ammonia by the method described in Patent Documents 4 and 5, and can also be used directly for various purification methods in accordance with respective environments.
That is, if fermentation solution contains ammonium salt/sodium salt, a purification method, which is most appropriate for worldwide various economical environments and climatic environments, can be selected.
Meanwhile, calcium is a relatively economical and reasonable neutralizer as described in Patent Document 3. As described above, magnesium, which allows only very restricted purification, is seldom evaluated as a neutralizer even if it is an essential metal ion for a microbial reaction.

[Patent Document 1] JP-A-02-283289
[Patent Document 2] US 6,284,904
[Patent Document 3] JP-A-03-030685
[Patent Document 4] JP-A-2001-514900
[Patent Document 5] US 5,958,744
[Patent Document 6] WO 98/01413

DISCLOSURE OF THE INVENTION

It is an object of the present invention to provide a method for producing organic acid ammonium solution efficiently.
In view of the above-mentioned circumstances, the inventors of the present invention have studied extensively in consideration that a method of purifying an organic acid could be most appropriately selected in various economical environments by converting organic acid magnesium salt into organic acid ammonium salt. As a result, the inventors of the present invention found that organic acid ammonium solution can be produced efficiently by culturing a microorganism in a medium added with a magnesium compound and subjecting the generated organic acid magnesium salt to salt-exchange by using an ammonia compound.
The inventors of the present invention further studied and found that magnesium carbonate containing substantially no ammonia can be obtained by heating magnesium carbonate/ammonium carbonate double salt which is formed as by-product, and thereby the organic acid ammonia salt can be produced with recycling the magnesium compound and without producing waste.
As described above, the inventors of the present invention have completed the present invention.
That is, the present invention is as follows.

(1) A method for producing solution of organic acid ammonium, which comprises a fermentation step of obtaining fermentation broth containing organic acid magnesium by using a microorganism having an organic acid-producing ability in the presence of a magnesium compound, a salt-exchanging step of subjecting the organic acid magnesium contained in said fermentation broth using an ammonium compound to produce organic acid ammonium and a magnesium compound, and a magnesium-separating step of separating the produced magnesium compound to obtain solution of organic acid ammonium.
(2) The method for producing solution of organic acid ammonium according to (1), wherein the magnesium compound separated in said magnesium-separating step is recycled as the magnesium compound in said fermentation step.
(3) The method for producing solution of organic acid ammonium according to (1) or (2), wherein the magnesium compound is magnesium carbonate and the ammonium compound is ammonium carbonate.
(4) The method for producing solution of organic acid ammonium according to any one of (1) to (3), wherein ammonium carbonate generated by providing carbon dioxide and ammonia into the fermentation broth is used as the ammonium compound.
(5) The method for producing solution of organic acid ammonium according to (4), wherein carbon dioxide is provided in a molar amount 0.3 to 10 times larger than that of magnesium in the fermentation broth.
(6) The method for producing solution of organic acid ammonium according to (4), further comprising the steps of heating the solution of organic acid ammonium obtained in said magnesium-separating step to vaporize and separate excess carbon dioxide and ammonia existing in the solution, and recycling the separated carbon dioxide and ammonia in said salt-exchanging step.
(7) The method for producing solution of organic acid ammonium according to (1), further comprising the steps of heating the magnesium carbonate separated in said magnesium-separating step to be decomposed into magnesium oxide and carbon dioxide, generating magnesium hydroxide by adding water into the magnesium oxide, and recycling the magnesium hydroxide as the magnesium compound in said fermentation step.
(8) The method for producing solution of organic acid ammonium according to (1), wherein said magnesium compound is magnesium hydroxide or a mixture of magnesium hydroxide and magnesium carbonate, and ammonia is used in said salt-exchanging step.

(9) The method for producing solution of organic acid ammonium according to (8), further comprising the steps of adding ammonium carbonate into the solution of organic acid ammonium obtained in said magnesium-separating step to further generate organic acid ammonium and magnesium carbonate, and obtaining solution of organic acid ammonium by separating the magnesium carbonate.

(10) The method for producing solution of organic acid ammonium according to any one of (1) to (9), wherein said salt-exchanging step is performed at the pH range between 7 and 12.

(11) The method for producing solution of organic acid ammonium according to any one of (1) to (10), wherein said organic acid is succinic acid and said organic acid ammonium is ammonium succinate.

(12) The method for producing solution of organic acid ammonium according to (1), further comprising the steps of heating or drying a double salt of magnesium carbonate and ammonium carbonate contained in the magnesium compound separated by said magnesium-separating step to remove ammonium carbonate and obtain magnesium carbonate, and recycling the magnesium carbonate in said fermentation step.

(13) The method for producing solution of organic acid ammonium according to (12), wherein ammonium carbonate is removed so that molar content of ammonia in said double salt becomes one tenth or lower with respect to that of magnesium.

(14) The method for producing solution of organic acid ammonium according to (12), wherein ammonium carbonate is removed so that molar content of ammonia in said double salt becomes one 30th or lower with respect to that of magnesium.

(15) The method for producing solution of organic acid ammonium according to (12), wherein said double salt is heated at the temperature of not less than 160°C.

BRIEF DESCRIPTION OF THE DRAWINGS

Fig. 1 shows the construction procedure and restriction map of the plasmid pKMB1.
Fig. 2 shows the construction procedure of the plasmid pKMB1/ΔLDH.
Fig. 3 shows the construction procedure of the plasmid pTZ4.
Fig. 4 shows the construction procedure of the plasmid pMJPC1.
Fig. 5 shows the construction procedure of the plasmid pFRPC1.1.

DESCRIPTION OF THE PREFERRED EMBODIMENTS

Hereinafter, the present invention will be described in detail.

The present invention relates to a method for producing solution of organic acid ammonium which comprises: a fermentation step of obtaining fermentation broth containing organic acid magnesium by using a microorganism having an organic acid-producing ability in the presence of a magnesium compound; a salt-exchange step of subjecting the organic acid magnesium contained in the fermentation broth using an ammonium compound to produce organic acid ammonium and a magnesium compound; and a magnesium-separation step of separating the produced magnesium compound to obtain solution of organic acid ammonium.

The kind of the organic acid ammonium is not particularly limited, as long as it is an ammonium salt of an organic acid that is produced by fermentation using a microorganism, but an ammonium salt of dicarboxylic acid or tricarboxylic acid is preferable. Examples of dicarboxylic acid include succinic acid, fumaric acid, maleic acid, malic acid, tartaric acid, asparaginic acid, glutaric acid, glutamic acid, adipic acid, suberic acid, itaconic acid, and terephthalic acid. An example of tricarboxylic acid includes citric acid.

In the present invention, the term "organic acid ammonium" includes organic acid monoammonium and organic acid multivalent ammonium.

In the fermentation step, the fermentation broth containing organic acid magnesium is obtained by using a microorganism having an organic acid-producing ability in the presence of a magnesium compound.

The microorganism to be used is a "microorganism having an organic acid-producing ability". The term "microorganism having an organic acid-producing ability" means a microorganism having an ability to produce and accumulate an organic acid in a medium when the microorganism is cultured in a medium containing a carbon source as described below. Examples of such microorganism include facultative anaerobic bacteria such as *Anaerobiospirillum* bacteria (US 5,143,833), *Actinobacillus* bacteria (US Patent 5,504,004), and *Escherichia* bacteria (description of US Patent No. 5,770,435), and aerobic bacteria such as coryneform bacteria (JP-A-11-113588) belonging to the genus *Brevibacterium*, the genus *Corynebacterium*, the genus *Arthrobacter*, or the like. A microorganism classified into *Corynebacterium glutamicum, Brevibacterium flavum, Brevibacterium ammoniagenes* or *Brevibacterium lactofermentum* is more preferable.

Specific examples of coryneform bacteria having succinic acid-producing ability include *Brevibacterium flavum* MJ233Δldh strain (JP-A-11-206385) in which lactate dehydrogenase activity is decreased, *Brevibacterium flavum* MJ233/pPCPYC strain (WO 01/27258) in which pyruvate carboxylase activity or phosphoenolpyruvate carboxylase activity is enhanced, *Brevibacterium flavum* MJ-233 (FERM BP-1497), *Brevibacterium flavum* MJ-233 AB-41 (FERM BP-1498), *Brevibacterium ammoniagenes* ATCC6872, *Corynebacterium glutamicum* ATCC31831, and *Brevibacterium lactofermentum* ATCC13869.

*Brevibacterium flavum* MJ-233 was deposited at the National Institute of Bioscience and Human-Technology, Agency of Industrial Science and Technology, Ministry of International Trade and Industry (current International Patent Organism Depositary, National Institute of Advanced Industrial Science and Technology) (Central 6, 1-1-1 Higashi, Tsukuba, Ibaraki, 305-8566, Japan) on April 28, 1975 with an accession number of FERM P-3068, and then transferred to the international deposit under the Budapest Treaty on May 1, 1981 and given an accession number of FERM BP-1497. *Brevibacterium ammoniagenes* ATCC6872 and the like can be distributed from the American Type Culture Collection (12301 Parklawn Drive, Rockville, Maryland 20852, United States of America).

The microorganism to be used in the fermentation step may be a microorganism modified so that the organic acid-producing ability is enhanced. Examples of a microorganism modified to have an enhanced succinic acid-producing ability include: a microorganism in which expression of pyruvate carboxylase gene is enhanced (JP-A-11-196888); and a microorganism in which lactate dehydrogenase gene is disrupted (JP-A-11-206385). Furthermore, a microorganism in which expression of fumarate reductase gene is enhanced as described in the below-mentioned Reference Examples can also be used.

The microorganism having an organic acid-producing ability to be used in the present invention is not limited to those as described above. In the present invention, other succinic acid-producing microorganisms may also be used, and the malic acid-producing microorganisms, fumaric acid-producing microorganisms, citric acid-producing microorganisms, isocitric acid-producing organisms, and the like which is obtainable by conventional methods may also be used.

The microorganism having an organic acid-producing ability to be used in the present invention may have an ability to produce two or more kinds of organic acids.

A liquid medium to be used for culture of the microorganism is a medium containing a carbon source. The carbon source is not particularly limited, as long as it can be assimilated by a microorganism. Examples of the carbon source include: carbohydrates such as galactose, lactose, glucose, maltose, fructose, glycerol, sucrose, saccharose, starch, and cellulose; and fermentable saccharides such as polyalcohols including glycerin, mannitol, xylitol, ribitol, and the like. Of those, glucose, fructose, or glycerol can be preferably used, and glucose can be particularly preferably used. In addition, cellulose that is a main component of paper can be preferably used as a raw material derived from a plant in a broad sense. Furthermore, a glycated starch liquid, molasses, or the like each containing the above-mentioned fermentable saccharide can also be used. Those carbon sources may be used alone or two or more kinds of them may be used in combination.

The concentration of the carbon source to be used is not particularly limited, but it is advantageous to increase the concentration as much as possible provided that the production of an organic acid is not inhibited. The carbon source is used within a concentration range of generally 5 to 30% (w/v), and preferably 10 to 20% (w/v). The carbon source may be supplementary added in accordance with decrease of the carbon source when the reaction progresses.

In addition to the carbon source, the liquid medium preferably contains a nitrogen source, an inorganic salt, and the like. The nitrogen source is not particularly limited, as long as it can be assimilated by the microorganism of the present invention to produce an organic acid. Specific examples thereof include various organic nitrogen compounds and inorganic nitrogen compounds such as an ammonium salt, nitrate, urea, soybean hydrolysate, casein hydrolysate, peptone, yeast extract, meat extract, and corn steepliquor. Phosphate salts, sulfate salts, and metal salts such as magnesium salt, potassium salt, manganese salt, iron salt, and zinc salt are used as an inorganic salt. In addition, to promote the growth of the microorganism, factors such as vitamins (for example, biotin, pantothenic acid, inositol, and nicotinic acid), nucleotides, and amino acids may be added if required. Furthermore, an appropriate amount of a commercially available anti-forming agent is preferably added in advance into a reaction aqueous solution to suppress foaming during the reaction.

For culture of the above-mentioned microorganism in such a liquid medium, a microorganism obtained by slant culture on a solid medium such as an agar medium may be used directly, or bacterial cells obtained by culture (seed culture) of the above-mentioned microorganism in advance in a liquid medium are preferably used. In this case, an amount of the bacterial cells to be used in the reaction is not particularly limited, but is generally 1 to 700 g/L, preferably 10 to 500 g/L, and more preferably 20 to 400 g/L.

As the fermentation reaction progresses, an organic acid is produced and pH of the culture solution decreases. Thus, a neutralizer is added to the culture solution. The neutralizer to be used is generally alkali earth metal compound, and preferably magnesium compound. The magnesium compound is advantageous because it increases the production of succinic acid and has a narrow pH fluctuation range. The magnesium compound to be used as a neutralizer preferably dissociates in an aqueous solution to be alkali, and examples thereof include magnesium hydroxide ($Mg(OH)_2$), magnesium carbonate ($MgCO_3$), and magnesium bicarbonate ($Mg(HCO_3)_2$). Of those, magnesium hydroxide is particularly preferable in view of easiness of pH adjustment. Two or more kinds of magnesium compounds may be used.

A manner of adding the magnesium compound is not particularly limited as long as the culture solution is controlled to have an appropriate pH. For example, the magnesium compound may be added as powder. The magnesium compound may be added to the medium at the start of culture or may be added during the culture. Alternatively, the magnesium compound may be added to the medium at the start of culture and further added during the culture if necessary. The pH value of the culture medium is adjusted with the magnesium compound within a range in which the microorganism exhibits an organic acid-producing ability most efficiently in accordance with the kind of the microorganism to be used. The pH value is adjusted to generally 4 to 10, and preferably about 6 to 9.

Culture conditions such as temperature and pressure in the fermentation step vary depending on the microorganism to be used, and conditions suitable for obtaining an organic acid may be selected for each case. For example, the culture temperature is generally 25°C to 40°C, and preferably 30°C to 37°C. The reaction time is preferably 1 hour to 168 hours, and more preferably 3 hours to 72 hours.

The fermentation broth after completion of the culture contains an organic acid magnesium which is composed of organic acid produced by the microorganism and magnesium in the neutralizer, which may be dissociated into organic acid ion and magnesium ion. The fermentation solution is preferably used in the salt-exchange step after the bacterial cells and the like have been removed by centrifugation or the like. The fermentation solution from which bacterial cells have been separated may be used in the salt-exchange step after being subjected to a purification operation. Furthermore, the fermentation broth may be used after being subjected to a concentration operation. The concentration operation may be performed by a conventional method, and a kettle reboiler or an evaporator may be used, for example. A multiple-effect evaporator may be used for mass production in which energy consumption is important.

The salt-exchange step is a step of subjecting the organic acid magnesium in the fermentation broth to salt-exchange by using an ammonia compound to produce organic acid ammonium and a magnesium compound such as magnesium carbonate, a double salt of magnesium carbonate, or magnesium hydroxide. The organic acid ammonium to be produced in this step may be dissociated into organic acid ion and ammonium ion.

Examples of the ammonia compound include ammonia and ammonium carbonate. Both of ammonia and ammonium carbonate may be added. A device to be used for the salt-exchange step may be a generally used crystallization tank such as a stirring tank, a draft tube, a crystal oslo-type crystallization tank, or a double propeller. The shape, method, and number of crystallization stage of the device are not particularly limited as long as it is a device capable of obtaining crystals by a solid-liquid equilibrium phenomenon.

Hereinafter, salt-exchange of magnesium succinate is explained. The salt-exchange of other organic acid magnesium may also be performed in the same manner as described below. Furthermore, reaction conditions may be changed appropriately within knowledge of a person skilled in the art.

First, salt-exchange by using ammonium carbonate is explained. The salt-exchange by using ammonium carbonate may be performed by adding ammonium carbonate (shown in the following reaction formula (I)) or by adding ammonia and carbon dioxide (shown in the following reaction formula (II)).

$$\text{Succinate-Mg}+(NH_4)_2CO_3 \rightarrow \text{Succinate}(NH_4)_2+MgCO_3\downarrow \qquad (I)$$

$$\text{Succinate-Mg}+2NH_3+CO_2+H_2O \rightarrow \text{Succinate}(NH_4)_2+MgCO_3\downarrow \qquad (II)$$

When ammonium carbonate is added, ammonium carbonate is added in an amount of 0.3 to 10 times mol, preferably 0.5 to 5 times mol, and more preferably 1 to 4 times mol with respect to the amount of magnesium in the fermentation broth to be supplied into the reaction tank. The phrase "magnesium in the fermentation broth" as used herein means a total amount of magnesium, which is contained in the magnesium succinate and the magnesium compound in the fermentation broth, and magnesium ion. When ammonia and carbon dioxide are added, an amount of carbon dioxide to be supplied to the reaction tank is 0.3 to 10 times mol, preferably 0.5 to 5 times mol, and more preferably 1 to 4 times mol with respect to the amount of magnesium in the fermentation broth to be supplied to the reaction tank. Ammonia is preferably added in an amount so that pH is in a range within which carbon dioxide maintains a predetermined solubility, that is, pH 7 to 12, more preferably 7.5 to 11, and particularly preferably 8 to 10. The time required for the salt-exchange reaction varies depending on a volume of magnesium succinate reaction solution and is not particularly limited, but is preferably 0.1 to 4 hours. The salt-exchange is performed under stirring in a pH range of preferably 7 to 12, more preferably 7.5 to 11, and much more preferably 8 to 10.

In the salt-exchange by using ammonium carbonate (reaction formula (I) or (II)), a recovery rate of magnesium is 90% or more, and yield of succinic acid is substantially equal to this value. Thus, ammonium succinate can be produced efficiently and magnesium can be recovered efficiently from the solution containing magnesium succinate.

Next, salt-exchange by using ammonia is explained. In this case, ammonium succinate and magnesium hydroxide are produced as shown in the following reaction formula (III).

$$Succinate\text{-}Mg+2NH_3+2H_2O \rightarrow Succinate(NH_4)_2+Mg(OH)_2\downarrow \quad (III)$$

An amount of ammonia to be added is preferably 2 to 15 times mol with respect to the amount of magnesium in the fermentation broth. The time required for the salt-exchange varies depending on the volume of the fermentation broth containing magnesium succinate and is not particularly limited, but is preferably 0.1 to 4 hours. The salt-exchange is performed in a pH range of preferably 7 to 12, more preferably 7.5 to 11, and much more preferably 8 to 10. In the salt-exchange by using ammonia, magnesium hydroxide is produced. Thus, the magnesium compound to be added to the fermentation broth is preferably magnesium hydroxide, or a mixture of magnesium hydroxide and magnesium carbonate.

In the salt-exchange by using ammonia (reaction formula (III)), a concentration of magnesium dissolved in aqueous ammonium succinate solution is about 0.5% wt. In order to increase a recovery rate, a volume of water as a solvent must be reduced, but the aqueous solution cannot be concentrated more than the solubility of ammonium succinate. In order to obtain aqueous ammonium succinate solution with a smaller magnesium concentration, ammonium carbonate is added to a filtrate obtained after the salt-exchange represented by the reaction formula (III), and then, salt-exchange represented by the reaction formula (I) or (II) is performed to produce and precipitate magnesium carbonate, followed by removal of the magnesium carbonate. Thereby, the magnesium concentration in the solution can be reduced to about 0.01 wt%.

The magnesium-separation step is a step of separating a magnesium compound such as the produced magnesium carbonate, a double salt of magnesium carbonate, or magnesium hydroxide, to obtain an organic acid ammonium solution. The magnesium compound hardly dissolves in water and thus can be removed by a conventional method such as filtration. For complete filtration of the magnesium compound, pressured filtration, vacuum filtration, centrifugal filtration, or the like may be used. Alternatively, the magnesium compound may be separated by centrifugation into a supernatant and a highly concentrated slurry, both of which are transferred by a pump. The magnesium compound is removed as described above, to thereby obtain aqueous organic acid ammonium solution.

The removed magnesium compound may be recycled as a neutralizer in the fermentation tank. It is economically preferable that the magnesium compound obtained in the magnesium-separation step is recycled as a magnesium compound in the fermentation step. In this case, heat sterilization is generally performed to prevent contamination of bacteria. In particular, when the magnesium compound is transferred as highly concentrated slurry, sterilization can be performed by heating with a general heat exchanger such as a multipipe heat exchanger or a plate type heat exchanger. Filtration may be performed after the heat sterilization, or the magnesium compound subjected to heat sterilization may be supplied to the fermentation tank directly. Even after complete filtration of the magnesium compound, steam may be applied directly to the magnesium compound for sterilization.

Magnesium carbonate may be recycled into carbon dioxide and magnesium hydroxide. That is, first, magnesium carbonate is decomposed by heat into carbon dioxide and magnesium oxide. The obtained carbon dioxide may be recycled to the salt-exchange reaction represented by the reaction formula (II). Meanwhile, magnesium oxide may be converted into magnesium hydroxide by a reaction with water and recycled to the first step as a magnesium-based neutralizer.

Magnesium carbonate is known to form a double salt under conditions in which a large concentration of carbon dioxide exists as described in JP-B-01-133919 or reveu de Chimie minerale t:22, 1985, p.692-698. Thus, when ammonium carbonate is used as an ammonium compound, a part of the magnesium compound produced in the salt-exchange step exists as a double salt of magnesium carbonate and ammonium carbonate. In this step, it is preferable that ammonium succinate containing as little magnesium as possible is recovered. However, reduction in amount of carbon dioxide surely increases a dissolved amount of magnesium based on a solubility product constant. Thus, formation of the double salt is inevitable. Meanwhile, magnesium is hardly recycled as the double salt as it is. Thus, slurry containing the double salt is separated, and then heated or dried, to thereby remove ammonium carbonate from the double salt and obtain magnesium carbonate. Then, the magnesium carbonate is preferably circulated to the fermentation step.

The magnesium compound containing the double salt is separated by a conventional method. For complete filtration of the magnesium compound, pressured filtration, vacuum filtration, centrifugal filtration, or the like may be used. Alternatively, the magnesium compound may be separated by centrifugation into supernatant and highly concentrated slurry, both of which are transferred by a pump. Preferably, the thus-obtained crystals or slurry is generally washed with ammonia or the like to remove organic substances, and then is supplied to a heating device for a subsequent heat operation.

The double salt containing magnesium carbonate and ammonium carbonate is heated, to thereby selectively decompose ammonium carbonate and obtain magnesium carbonate with high purity. The heating temperature is preferably 108°C to 210°C, and more preferably 120°C to 180°C. The heating time varies depending on the amount of the double salt and the heating device and is not particularly limited, but is preferably 15 minutes to 2 hours, and more preferably 30 minutes to 1 hour. Magnesium carbonate to be obtained by heating the double salt need not have a purity of 100% and may contain a trace amount of remaining ammonium carbonate. In this case, an amount of ammonia in the double salt is preferably not more than 1/10 times mol, and more preferably not more than 1/30 times mol with respect to the amount of magnesium in the double salt.

The heating device to be used may be any type as long as it is capable of heating crystals to a temperature exceeding a predetermined temperature. Examples of the heating device include a kiln, a drier, and a heater. When the crystals may be flaky, a hot plate, a belt-type heater, or a baking furnace may be used. In general, a neutralizer is preferably in a form of a powder because of good dispersibility and easy handling. In this case, a rotary kiln or a fluidized drier is preferably used.

When the double salt, which is formed by salt-exchange of organic acid magnesium by using ammonium carbonate, is heated, the double salt is preferably washed with aqueous ammonia for removing an organic acid from the double salt and then heated. The concentration of aqueous ammonia is not particularly limited, and 25% industrial aqueous ammonia or the like may be used, for example. The washing may be performed once or several times.

Magnesium carbonate to be recovered after ammonium carbonate has been removed from the double salt by heating may be recycled, to thereby improve the efficiency of organic acid fermentation production. That is, magnesium carbonate may be recycled as a neutralizer in the fermentation step. Alternatively, as described above, magnesium carbonate may be converted into magnesium hydroxide and carbon dioxide, and the magnesium hydroxide and carbon dioxide may be recycled in the fermentation step and the salt-exchange step, respectively.

Meanwhile, the organic acid ammonium solution obtained from the magnesium-separation step may contain unreacted carbon dioxide (existing as carbonate ion ($HCO_3^-$) in the solution) which had been added excessively, and ammonia. In this case, carbon dioxide and ammonia can be separated easily by evaporation and vaporization by heating based on the property that the solubilities of carbon dioxide and ammonia are highly dependent on temperature. In this case, carbon dioxide and ammonia are vaporized at the same time, and thus may be precipitated as ammonium carbonate upon cooling and may cause clogging of a reaction tank. Thus, a temperature of the vaporized gas is preferably higher than the melting point of ammonium carbonate of 108°C. However, even if the temperature is lower than 108°C, ammonium carbonate can be removed sufficiently at 80°C or higher, and preferably 90°C at normal pressure, for example. At 80°C, a sufficient volume of water vapor is often not obtained at normal pressure, and clogging may occur depending on a level of cooling. Even in this case, the clogging will not occur if some volume of steam is allowed to coexist by controlling the pressure. Thus, at 108°C or lower, a volume of the coexisting water must be controlled by pressure. A particularly preferable condition comprises heating the aqueous organic acid ammonium solution to 108°C or higher upon vaporization, that is, to 108°C or higher to vaporize carbonic acid and ammonia and allowing enough volume of water to coexist.

An example of a safe method of recovering and recycling vaporized carbonic acid and ammonia comprises: absorbing carbonic acid and ammonia by using a sufficient volume of water to dissolve the total amount of them as ammonium carbonate; storing the ammonium carbonate in a buffer tank; and supplying the ammonium carbonate from the buffer tank to a salt-exchange reaction tank. However, in this method, water which is required for dissolving ammonium carbonate is supplied to the salt-exchange reaction tank at the same time. Thus, carbonic acid and ammonia are preferably recycled to the salt-exchange reaction tank as a gas from the viewpoint of energy-saving. At 108°C or higher, carbonic acid and ammonia separated from the aqueous organic acid ammonium solution are supplied to the salt-exchange reaction tank as a slightly pressured gas.

The organic acid ammonium obtained by the method of the present invention may be used to obtain an organic acid. The method of obtaining the organic acid from the organic acid ammonium is not particularly limited. Examples thereof include: a method using electrodialysis (JP-A-02-283289); a method using an ion-exchange resin (US 6,284,904 or WO01/66508); a method of decomposing an organic acid calcium, which is produced by fermentation with neutralization with calcium hydroxide, with sulfuric acid (JP-A-03-030685); a method of performing reactive crystallization by a salt-exchange reaction with sulfuric acid (JP-A-2001-514900 or US 5,958,744); a reactive extraction method (WO 98/01413); and a method using acetic acid (WO 03/95409).

EXAMPLES

Hereinafter, the present invention is described in more detail with reference to examples. In the examples, production of ammonium succinate solution is described, but solution of other organic acid magnesium can be produced in the same manner as described below.

First, the salt-exchange step and following steps are described in Examples 1 and 2. Example 1: Salt-exchange by using ammonia

(1) Preparation of Mg succinate solution (Mg succinate concentration 12.3 wt%, 30 kg)

25.35 kg of distilled water was added into 100L stirring vessel and stirred at 30 rpm with an anchor blade. The stirring vessel was adjusted to normal temperature by passing water through a jacket. 1.54 kg of magnesium hydroxide (Wako Pure Chemical Industries, Ltd.) was added into the stirring tank, to thereby prepare a suspension. 3.12 kg of succinic acid (Wako Pure Chemical Industries, Ltd.) was gradually added to this suspension while temperature of the suspension was monitored. This neutralization reaction provided 30 kg of aqueous magnesium succinate solution.

(2) Salt-exchange reaction by using aqueous ammonia

Following the above-mentioned operation, 25% aqueous ammonia (Wako Pure Chemical Industries, Ltd.) was mixed into the aqueous magnesium succinate solution (30 kg) for salt-exchange. The mixing method was as follows: 15 kg of aqueous ammonia (25%) was added over about 1 hour; and after completion of the addition, the mixture was stirred for 60 minutes to complete the salt-exchange reaction. Thus, slurry (45 kg) containing precipitated magnesium hydroxide was obtained. A small volume of this slurry was sampled, and the sample was filtered through 0.2 μm membrane filter (Millipore Corporation). The thus-obtained filtrate containing ammonium succinate was analyzed for its Mg concentration by means of ion chromatography (electric conductivity detector). As a result, the Mg concentration was 0.39 wt%.

(Charged Mg amount) $2.14(wt\%)/100\times30(kg)=0.642(kg)$

(Mg amount in the supernatant) $0.39(wt\%)/100\times45(kg)=0.176(kg)$

(Mg removal rate) $(1-0.176/0.642)\times100=72.6(\%)$

(3) Solid-liquid separation of slurry with screw decanter

The slurry obtained by the above-mentioned operation was subjected to solid-liquid separation. A screw decanter (Sharpless Super Decanter P-660, manufactured by Tomoe Engineering Co., Ltd.) was used for the solid-liquid separation. The inner cylinder and outer cylinder of the decanter were set to 3,900 rpm and 5,100 rpm, respectively. Under the condition, the slurry was allowed to flow through the decanter at 30 L per hour for continuous solid-liquid separation. It was observed that a liquid discharged from the liquid discharge port contained a slight amount of powder. A part of this liquid was sampled and a small amount of tartaric acid was added thereto for dissolving the powder, to thereby analyze the Mg concentration. As a result, the Mg concentration was 0.49 wt%. The discharged solid was analyzed for its Mg concentration in the same manner as described above. The solid was diluted 50 times with distilled water, and tartaric acid was added thereto until the solid was completely dissolved, to thereby prepare a uniform solution. As a result of the analysis of this solution, the Mg concentration was 14.6 wt%. Furthermore, the recovered liquid and the recovered solid were analyzed for succinic acid concentration. As a result, the succinic acid concentration was 6.79% and 7.89%, respectively. Ultimately, the amount of the liquid recovered by the solid-liquid separation was 42 kg, and the amount of the recovered solid was 3 kg.

(Amount of Mg in the recovered liquid) $0.49(wt\%)/100\times42(kg)=0.206(kg)$

(Amount of Mg in the recovered solid) $14.6(wt\%)/100\times3(kg)=0.438(kg)$

(Mg recovery rate with respect to the charged Mg amount) $(1-0.206/0.642)\times100=67.9(\%)$

(Amount of succinic acid in the recovered liquid) 6.79(wt%)/100×42(kg)=2.85(kg)

(Amount of succinic acid in the recovered solid) 7.89(wt%)/100×3(kg)=0.24(kg)

(Succinic acid recovery rate with respect to the charged succinic acid amount)

2.85/3.12×100=91.5%

Example 2: Salt-exchange by using ammonium carbonate

(1) Synthesis of Mg succinate solution (Mg succinate concentration 12.3 wt%, 10 kg)

8.45 kg of distilled water was added into 15L stirring vessel and stirred at 200 rpm with an inclined paddle blade. The stirring vessel was adjusted at normal temperature by passing water through a jacket. 0.51 kg of magnesium hydroxide (Wako Pure Chemical Industries, Ltd.) was added into a stirring tank, to thereby prepare a suspension. 1.04 kg of succinic acid (Wako Pure Chemical Industries, Ltd.) was gradually added into this suspension while the temperature of the suspension was monitored. This neutralization reaction provided 10 kg of aqueous magnesium succinate solution.

(2) Salt-exchange reaction by using ammonia carbonate

Following the above-mentioned operation, ammonia carbonate (Wako Pure Chemical Industries, Ltd.) was mixed into the aqueous magnesium succinate solution (10 kg) for salt-exchange. The mixing method was as follows: 2.1 kg of ammonia carbonate was added over about 1 hour; and after completion of the addition, the mixture was stirred for 60 minutes to complete the salt-exchange reaction. Thus, slurry (12.1 kg) containing precipitated magnesium hydroxide was obtained. A small volume of this slurry was sampled, and the sample was filtered through a 0.2 μm membrane filter (available from Millipore Corporation). The thus-obtained filtrate which contains ammonium succinate was analyzed for its Mg concentration by means of ion chromatography (electric conductivity detector). As a result, the Mg concentration was 0.01 wt%.

(Charged Mg amount) 2.14(wt%)/100×10(kg)=0.214(kg)

(Mg amount in the supernatant) 0.01(wt%)/100×12.1(kg)=0.0012(kg)

(Mg removal rate) (1-0.0012/0.214)×100=99.4(%)

(3) Solid-liquid separation with pressure filter

The slurry obtained by the above-mentioned operation was subjected to solid-liquid separation. A 10-L pressure filter (Advantech Co., Ltd.) was used for the solid-liquid separation. A high purity filter No. 5C (Advantech Co., Ltd.) was used as a filter. Continuous filtration was performed twice. Filtration was performed at a gauge pressure of 4 kg, and the pressure was returned to a normal pressure when no more liquid was discharged. Then, another slurry was introduced. Under such a condition, the slurry was subjected to the solid-liquid separation. No solid content was observed in the recovered filtrate. This filtrate was subjected to Mg analysis, resulting in the Mg concentration of 0.01 wt%. The recovered solid was analyzed for Mg concentration in the same manner as described above. The solid was diluted 50 times with distilled water, and tartaric acid was added thereto until the solid was completely dissolved, to thereby prepare a uniform solution. As a result of the analysis of this solution, the Mg concentration was 7.48 wt%. Furthermore, the recovered liquid and the recovered solid were analyzed for the succinic acid concentration. As a result, the succinic acid concentration was 10.1 % and 4.4%, respectively. Ultimately, the amount of the liquid recovered by the solid-liquid separation was 9.1 kg, and the amount of the recovered solid was 2.8 kg.

(Amount of Mg in the recovered liquid) $0.01(wt\%)/100\times 9.1(kg)=0.00091(kg)$

(Amount of Mg in the recovered solid) $7.48(wt\%)/100\times 2.8(kg)=0.209(kg)$

(Mg recovery rate with respect to the charged Mg amount)

$$(1-0.00091/0.214)\times 100=99.6(\%)$$

(Amount of succinic acid in the recovered liquid) $10.1(wt\%)/100\times 9.1(kg)=0.919(kg)$

(Amount of succinic acid in the recovered solid) $4.4(wt\%)/100\times 2.8(kg)=0.123(kg)$

(Succinic acid recovery rate with respect to the charged succinic acid amount)

$$0.919/1.04\times 100=88.4\%$$

Ammonium succinate solution was obtained by recovering magnesium from magnesium succinate by means of the salt-exchange with ammonia or the salt-exchange with ammonium carbonate. Magnesium could be recovered more efficiently and substantially completely by the salt-exchange with ammonium carbonate as compared with the salt-exchange with ammonia.

The following Example 3 describes the fermentation step, the salt-exchange step, the magnesium-separation step, and the step of obtaining magnesium carbonate from a magnesium carbonate/ammonium carbonate double salt obtained in the magnesium-separation step. The Example 4 describes the recycling of magnesium carbonate obtained from the double salt in the fermentation step.

<Example 3: Preparation of magnesium succinate solution by fermentation and preparation of the double salt>

(1) Preparation of bacterial cells

100 mL of a medium, which contains 4 g of urea, 14 g of ammonium sulfate, 0.5 g of monopotassium phosphate, 0.5 g of dipotassium phosphate, 0.5 g of magnesium sulfate heptahydrate, 20 mg of ferrous sulfate heptahydrate, 20 mg of manganese sulfate hydrate, 200 μg of D-biotin, 200 μg of thiamine chloride, 1 g of yeast extract, 1 g of casamino acid in 1,000 mL of distilled water, was added into 500-mL Erlenmeyer flask, and the medium was subjected to heat sterilization at 120°C for 20 minutes, followed by cooling to room temperature. Then, 4 mL of 50% aqueous glucose solution sterilized in advance and 50 μL of 5% kanamycin solution sterilized by filtration were added thereto. *Brevibacterium flavum* MJ233/FRD/PC/ALDH strain which was constructed in the Reference Example as described below was inoculated therein and subjected to seed culture at 30°C for 24 hours. In this strain, expression of fumarate reductase gene and pyruvate carboxylase gene has been enhanced and lactate dehydrogenase gene has been disrupted.

A medium, which contains 12 g of urea, 42 g of ammonium sulfate, 1.5 g of monopotassium phosphate, 1.5 g of dipotassium phosphate, 1.5 g of magnesium sulfate heptahydrate, 60 mg of ferrous sulfate heptahydrate, 60 mg of manganese sulfate hydrate, 600μ g of D-biotin, 600 μg of thiamine chloride, 3 g of yeast extract, 3 g of casamino acid, and 1 mL of anti-foaming agent (Adekanol LG294, available from Asahi Denka Co., Ltd.) in 2,500 mL of distilled water, was added into 5-L fermentation tank, and the medium was subjected to heat sterilization at 120°C for 20 minutes, followed by cooling to room temperature. Then, 500 mL of 12% aqueous glucose solution sterilized in advance was added thereto. The total amount of the above-mentioned seed culture solution was added thereto, and maintained at 30°C. The culture was performed at an aeration rate of 500 mL per minute and a stirring rate of 500 rpm. After 12 hours, glucose was substantially consumed. This broth was subjected to centrifugation at 8,000 rpm for 5 minutes, and a supernatant was removed, to thereby obtain bacterial cells to be used for the following fermentation reaction.

(2) Fermentation reaction

A medium, which contains 0.36 g of monopotassium phosphate, 0.36 of dipotassium phosphate, 1.8 g of magnesium sulfate heptahydrate, 72 mg of ferrous sulfate heptahydrate, 72 mg of manganese sulfate hydrate, 720 μg of D-biotin, and 720 μg of thiamine chloride in 2,600 mL of distilled water, was added into a 5-L jar, and the medium was subjected to heat sterilization at 120°C for 20 minutes, followed by cooling to room temperature. Then, the medium was added to the bacterial cells which had been collected from the culture to resuspend the cells so that O.D. (660 nm) was 60. 2,600 mL of this suspension and 1,000 mL of 36% aqueous glucose solution sterilized in advance were added into a 5-L jar, and 349 g of $4MgCO_3 \cdot Mg(OH)_2 \cdot 5H_2O$ and 27 g of ammonium succinate were added thereto and mixed. This reaction suspension was maintained at 35°C, and the reaction was performed while stirring at 300 rpm. After completion of the fermentation, the culture broth was subjected to centrifugation at 8,000 rpm for 5 minutes. The supernatant was obtained as a fermentation broth, and the precipitated bacterial cells were recovered. The fermentation reaction and the separation operation were performed six times in the same way by using the recovered bacterial cells, to thereby obtain 20 L of supernatant in total. In the six times operations, the average accumulation amount of succinic acid was 89.5 g/L and the average yield of succinic acid was 82%. Furthermore, the supernatant was filtered by using a UF membrane with a molecular cutoff weight of 20,000, to thereby obtain a fermentation broth containing magnesium succinate.

(3) Salt-exchange

20 L of the aqueous magnesium succinate solution obtained by the fermentation was added into a 50L stirring tank, and 4.0 kg of ammonium carbonate (special grade, Wako Pure Chemical Industries, Ltd.) was added thereto under a normal temperature and a normal pressure. The mixture was stirred, and crystal was precipitated immediately. After stirring for 120 minutes, the mixture was subjected to centrifugation by using a screw decanter (3,000 G, Tomoe Engineering, Inc.), to thereby obtain 19.9 kg of ammonium succinate solution and 4.1 kg of magnesium carbonate/ammonium carbonate double salt.

(4) Decomposition of the double salt

The double salt obtained as described above was sampled and was subjected to washing and heating as described below. Studies were conducted by varying the number of washing, heating temperature, and heating time. Table 1 shows the conditions including temperature, time, and number of washing for each experimental example.

(Washing)

The magnesium carbonate/ammonium carbonate double salt obtained by the Solvay salt-exchange was sampled into a beaker, and 25% industrial aqueous ammonia (Mitsubishi Chemical Corporation) of an equal weight to that of the double salt was added thereto for washing in suspension. The obtained suspension was subjected to suction filtration with a Nutsche for solid-liquid separation, to thereby recover a solid. The washing and filtration operation was performed once or twice.

(Heating)

The solid obtained by the washing operation was added into a round bottom flask of a total volume of 500 mL, and heated at a predetermined temperature and for a predetermined time by using a rotary evaporator. Amounts of Mg, ammonia, and succinic acid in the obtained heated solid were analyzed.

Table 1 shows the results of the analysis of the obtained heated solid. It was revealed from Experiment Nos. 1 to 6 that, when the double salt was heated at 120°C to 180°C, the ratio of ammonia in the double salt decreased significantly and the ratio of magnesium increased. This result indicated that ammonium carbonate was efficiently removed from the double salt and magnesium carbonate with high purity was obtained. Meanwhile, when the double salt was heated at 90°C (Experiment No. 7), a substantial amount of ammonia remained in the double salt and ammonium carbonate was not sufficiently removed. Succinic acid adhered to the double salt was removed by washing with 25% ammonia water, and succinic acid was removed more efficiently by washing twice, in the Examples.

Table 1

| Experiment No. | Raw material | 1 | 2 | 3 | 4 | 5 | 6 | 7 |
|---|---|---|---|---|---|---|---|---|
| Salt-exchanged cake (g) | 300 | 300 | 300 | 300 | 300 | 300 | 300 | 300 |
| 25% aqueous ammonium (g) | 0 | 300 | 300 | 300 | 300 | 300 | 300 | 300 |
| Number of washing | 0 | 1 | 2 | 1 | 2 | 1 | 2 | 2 |
| Heating temperature (°C) | - | 140 | 140 | 160 | 160 | 180 | 120 | 90 |
| Heating time (min) | 0 | 42 | 60 | 60 | 60 | 60 | 60 | 60 |
| | | | | | | | | |
| Mg concentration (wt%) | 10.39 | 17.73 | 24.26 | 25.94 | 28.02 | 30.78 | 18.07 | 10.89 |
| $NH_3$ concentration (wt%) | 11.35 | 0.53 | 0.22 | 0.23 | 0.23 | 0.23 | 0.16 | 7.15 |
| Succinate concentration (wt%) | 1.55 | 0.97 | 0.59 | 1.33 | 0.65 | 1.44 | 0.38 | 0.22 |
| Acetate concentration (wt%) | 0.151 | 0.084 | 0.037 | 0.136 | 0.047 | 0.126 | 0.02 | 0.002 |
| | | | | | | | | |
| Recovery (g) | 300 | 168.7 | 104 | 107.6 | 102.8 | 90.8 | 170.8 | 255.8 |
| | | | | | | | | |
| Mg (mol) | 1.28 | 1.23 | 1.04 | 1.15 | 1.19 | 1.15 | 1.27 | 1.15 |
| $NH_3$ (mol) | 2.00 | 0.05 | 0.01 | 0.01 | 0.01 | 0.01 | 0.02 | 1.08 |
| Succinate (mol) | 0.04 | 0.014 | 0.01 | 0.012 | 0.006 | 0.011 | 0.01 | 0.005 |
| Acetate (mol) | 0.0075 | 0.0024 | 0.0006 | 0.0024 | 0.0008 | 0.0019 | 0.0006 | 0.0001 |
| | | | | | | | | |
| $NH_3$/Mg (mol/mol) | 1.56 | 0.04 | 0.01 | 0.01 | 0.01 | 0.01 | 0.01 | 0.94 |
| Succinate/Mg (mol/mol) | 0.031 | 0.011 | 0.005 | 0.011 | 0.005 | 0.010 | 0.004 | 0.004 |

Example 4: Recycle of magnesium carbonate obtained by decomposition of the double salt in the fermentation step

First, the bacterial cells to be used for the fermentation reaction were obtained by the method (1) of Example 3. Next, the fermentation reaction was performed by using as a neutralizer magnesium carbonate obtained in the step of decomposing the double salt in Example 3. A medium, which contains 0.04 g of monopotassium phosphate, 0.04 g of dipotassium phosphate, 0.2 g of magnesium sulfate heptahydrate, 8 mg of ferrous sulfate heptahydrate, 8 mg of manganese sulfate hydrate, 80 $\mu$g of D-biotin, and 80 $\mu$g of thiamine chloride in 200 mL of distilled water, was introduced into a 500mL Erlenmeyer flask, and the medium was subjected to heat sterilization at 120°C for 20 minutes, followed by cooling to room temperature. Then, the medium was added to the bacterial cells collected from the culture to resuspend the cells so that O.D. (660 nm) was 60. 200 mL of this suspension and 200 mL of 20% aqueous glucose solution sterilized in advance were added into a 1-L jar, and 42 g of magnesium carbonate (one obtained in Section (4) of Example 1 or commercially available one) and 3 g of ammonium succinate were added thereto and mixed. This reaction suspension was maintained at 35°C, and the reaction was performed while stirring at 400 rpm. 9 hours after the start of the reaction, glucose was substantially consumed. The accumulation amount of succinic acid was 91 g/L, and yield thereof was 83%.

Table 2 shows the results. Experiment Nos. 1, 2, 4, and 5 in Table 2 show the results of the experiments of the above-mentioned fermentation production of succinic acid in which magnesium carbonate obtained in the corresponding Experiment Nos. in Table 1 was recycled as a neutralizer. Reagent 1 and Reagent 2 show the results of the experiments of the above-mentioned fermentation production of succinic acid in which commercially available magnesium carbonate was used. The results of the reaction indicate that, when the fermentation production of succinic acid was performed by using magnesium carbonate obtained by heating the double salt, a substantially equal amount of succinic acid could be obtained as compared with the experiment in which commercially available magnesium carbonate was used. It was revealed from the results that magnesium carbonate obtained by heating the double salt could be recycled efficiently.

Table 2

| Experiment No. | Raw material | 1 | 2 | Reagent 1 | 4 | 5 | Reagent 2 |
|---|---|---|---|---|---|---|---|
| Salt-exchanged cake (g) | 300 | 300 | 300 | | 300 | 300 | |
| 25% aqueous ammonium (g) | 0 | 300 | 300 | | 300 | 300 | |
| Number of washing | Without washing | 1 | 2 | | 2 | 1 | |
| Heating temperature (°C) | - | 140 | 140 | | 160 | 180 | |
| Heating time (min) | Without heating | 42 | 60 | | 60 | 60 | |
| Analysis of magnesium carbonate | | | | | | | |
| Mg concentration (wt%) | 10.39 | 17.73 | 24.26 | | 28.02 | 30.78 | |
| $NH_3$ concentration (wt%) | 11.35 | 0.53 | 0.22 | | 0.23 | 0.23 | |
| Succinate concentration (wt%) | | 1.04 | 0.63 | | 0.69 | | |
| $NH_3$/Mg (mol%) | | 4.25 | 1.28 | | 1.14 | 1.07 | |
| Results of fermentation reaction | | | | | | | |
| Succinate yield (%) | | 83.25 | 92.56 | 89.52 | 88.90 | 90.01 | 89.60 |
| Reaction rate (g/gdcw/hr) | | 0.41 | 0.41 | 0.23 | 0.35 | 0.33 | 0.25 |
| Acetate/Succinate (wt%) | | 19.21 | 18.84 | 16.67 | 18.22 | 16.09 | 15.74 |

Reference Examples

The methods of constructing a microorganism used in the fermentation reaction in the Examples 3 and 4 are described.

Reference Example 1: Construction of a gene disruption vector

(A) Extraction *of Bacillus subtilis* genomic DNA

*Bacillus subtilis* ISW1214 was cultured until a late logarithmic growth phase in a 10mL of LB medium [composition: 10 g of tryptone, 5 g of yeast extract, and 5 g of NaCl dissolved in 1 L of distilled water], and the bacterial cells were collected. The obtained bacterial cells were suspended in 0.15 mL of 10 mM NaCl/20 mM Tris buffer (pH of 8.0)/ 1 mM EDTA·2Na containing 10 mg/mL of lysozyme. Then, proteinase K was added to the suspension at a final concentration of 100μg/mL, and maintained at 37°C for 1 hour. Then, sodium dodecyl sulfate solution was added thereto at a final concentration of 0.5%, and maintained at 50°C for 6 hours for lysis. To this lysate, an equal amount of a phenol/ chloroform solution was added, and shaken slowly at room temperature for 10 minutes. Then, the total suspension was subjected to centrifugation (5,000×g, 20 minutes, 10 to 12°C), and a supernatant fraction was taken. Sodium acetate solution was added to the supernatant fraction at a concentration of 0.3 M, and then twice amount of ethanol was added and mixed. A precipitate was recovered by centrifugation (15,000×g, 2 minutes), then washed with 70% ethanol and air dried. 5 mL of 10mM Tris buffer (pH of 7.5)/1 mM EDTA·2Na was added to the obtained DNA. The resultant solution was left standing overnight at 4°C, and used as a template DNA for PCR.

(B) Amplification and cloning of SacB gene by PCR

*A Bacillus subtilis* SacB gene was obtained by performing PCR by using the DNA prepared in the above section (A) as a template; and using synthetic DNAs (SEQ ID NOS: 1 and 2) designed based on the reported nucleotide sequence of the gene (GenBank Database AccessionNo. X02730).

The composition of the reaction solution is as follows. 1 μL of the template DNA, 0.2 μL of PfxDNA polymerase (available from Invitrogen), 1-fold concentration of the supplied buffer, 0.3 μM of respective primers, 1 mM $MgSO_4$,

and 0.25 μM dNTPs were mixed, and total volume of the reaction solution was adjusted to 20 μL.
Reaction temperature condition is as follows: The DNA Thermal Cycler PTC-2000 manufactured by MJ Research Co., Ltd. was used and a cycle of 94°C for 20 seconds and 68°C for 2 minutes was repeated 35 times. For the first cycle, heat-retention at 94°C was conducted for 1 minute 20 seconds. For the last cycle, the heat-retention at 68°C was conducted for 5 minutes.
An amplified product was analyzed by separating it in 0.75% agarose (SeaKem GTG agarose, available from FMC BioProducts) gel electrophoresis and visualizing with ethidium bromide staining, to thereby detect a fragment of about 2 kb. The target DNA fragment was recovered from the gel by using QIAQuick Gel Extraction Kit (available from QIAGEN).
A 5'-end of the recovered DNA fragment was phosphorylated with T4 Polynucleotide Kinase (available from Takara Shuzo Co., Ltd.) and was inserted into an EcoRV site of the *Escherichia coli* vector (pBluescript II: available from STRATEGENE) by using Ligation Kit ver. 2 (available from Takara Shuzo Co., Ltd.), and the obtained plasmid DNA was used to transform *Escherichia coli* (DH5α strain). The obtained recombinant *Escherichia coli* was spread over an LB agar medium (10 g of tryptone, 5 g of yeast extract, 5 g of NaCl, and 15 g of agar dissolved in 1 L of distilled water) containing 50 μg/mL ampicillin and 50 μg/mL X-Gal.
Clones each forming a white colony on this medium were transferred to an LB agar medium containing 50 μg/mL ampicillin and 10% sucrose, and was cultured at 37°C for 24 hours. Of those clones, clones which could not grow on the medium containing sucrose were subjected to liquid culture by a conventional method, and then the plasmid DNA was isolated. An *Escherichia coli* strain in which SacB gene is functionally expressed must be incapable of growing in the medium containing sucrose. The obtained plasmid DNA was digested with restriction enzymes SalI and PstI. The plasmid DNA was confirmed to have an insert of about 2 kb and the plasmid was named pBS/SacB.

(C) Construction of chloramphenicol-resistant SacB vector
500 ng of *Escherichia coli* plasmid vector pHSG396 (chloramphenicol resistant marker, available from Takara Shuzo Co., Ltd.) was reacted with 10 units of restriction enzyme PshBI at 37°C for 1 hour, and recovered by phenol/chloroform extraction and ethanol precipitation. Both ends of the resultant DNA were each made blunt with Klenow Fragment (available from Takara Shuzo Co., Ltd.), and MluI linker (available from Takara Shuzo Co., Ltd.) was ligated thereto by using the Ligation Kit ver. 2 (available from Takara Shuzo Co., Ltd.) to form a circular plasmid, and the obtained plasmid was used to transform the *Escherichia coli* (DH5α strain). The obtained recombinant *Escherichia coli* was spread on an LB agar medium containing 34 μg/mL chloramphenicol. A plasmid DNA was isolated from the obtained clones by a conventional method. A clone having a cleavage site of a restriction enzyme MluI was selected and named pHSG396Mlu.
Meanwhile, pBS/SacB constructed in the above section (B) was digested with the restriction enzymes SalI and PstI, and both ends of the obtained DNA were each made blunt with the Klenow Fragment. The MluI linker was ligated thereto by using the Ligation Kit ver. 2 (available from Takara Shuzo Co., Ltd.). Then, a DNA fragment of about 2.0 kb containing SacB gene was separated in 0.75% agarose gel electrophoresis, and recovered. This SacB gene fragment was ligated to the fragment obtained by digesting pHSG396Mlu with the restriction enzyme MluI and dephosphorylated with Alkaline Phosphatase Calf intestine (available from Takara Shuzo Co., Ltd.), by using the Ligation Kit ver. 2 (available from Takara Shuzo Co., Ltd.), and the obtained DNA was used to transform the *Escherichia coli* (DH5α strain). The obtained recombinant *Escherichia coli* was spread on an LB agar medium containing 34μg/mL chloramphenicol.
The obtained colonies were transferred to an LB agar medium containing 34μg/mL chloramphenicol and 10% sucrose, and cultured at 37°C for 24 hours. Among these clones, plasmid DNA was isolated from the clones which could not grow on the medium containing sucrose by a conventional method. The obtained plasmid DNA was subjected to MluI digestion and analyzed. As a result, the plasmid DNA was confirmed to have an insert of about 2.0 kb and named pCMB1.

(D) Acquisition of kanamycin-resistant gene
A kanamycin-resistant gene was obtained by performing PCR using a DNA of *Escherichia coli* plasmid vector pHSG299 (kanamycin resistant marker, Takara Shuzo Co., Ltd.) as a template; and using synthetic DNAs (shown in SEQ ID NOS: 3 and 4) as primers. The composition of the reaction solution is as follows: 1 ng of the template DNA, 0.1 μL of Pyrobest DNA polymerase (available from Takara Shuzo Co., Ltd.), 1-fold concentration of the supplied buffer, 0.5 μM of respective primers, and 0.25 μM dNTPs were mixed, and a total volume of the reaction solution was adjusted to 20 μL.

Reaction temperature condition is as follows: The DNA Thermal Cycler PTC-2000 manufactured by MJ Research Co., Ltd. was used and a cycle of 94°C for 20 seconds, 62°C for 15 seconds, and 72°C for 1 minute 20 seconds was repeated 20 times. For the first cycle, heat-retention at 94°C was conducted for 1 minute 20 seconds. For the last cycle, the heat-retention at 72°C was conducted for 5 minutes.
An amplified product was analyzed by separating in 0.75% agarose (SeaKem GTG agarose, available from FMC BioProducts) gel electrophoresis and visualizing with ethidium bromide staining, to thereby detect a fragment of about 1.1 kb. The target DNA fragment was recovered from the gel by using the QIAQuick Gel Extraction Kit (available from QIAGEN). A 5'-end of the recovered DNA fragment was phosphorylated with T4 Polynucleotide Kinase (available from Takara Shuzo Co., Ltd.).

(E) Construction of kanamycin-resistant SacB vector
A DNA fragment of about 3.5 kb obtained by digesting pCMB1 constructed in the above section (C) with restriction enzymes Van91I and ScaI was separated in 0.75% agarose gel electrophoresis, and recovered. The resultant DNA was mixed with the kanamycin resistant gene prepared in the above section (D) and ligated thereto by using the Ligation Kit ver. 2 (available from Takara Shuzo Co., Ltd.), and the obtained plasmid DNA was used to transform the *Escherichia coli* (DH5α strain). The obtained recombinant *Escherichia coli* was spread over an LB agar medium containing 50 μg/mL kanamycin.
A strain grown on the medium containing kanamycin was confirmed to be incapable of growing on the medium containing sucrose. Furthermore, the plasmid DNA prepared from the same strain showed the fragments of 354, 473, 1,807, and 1,997bp by restriction enzyme HindIII digestion. Thus, it was concluded that the plasmid has the structure shown in Fig. 1, and the plasmid was named pKMB1.

Reference Example 2: Construction of a LDH gene-disrupted strain

(A) Extraction of a genomic DNA of *Brevibacterium flavum* MJ233-ES strain

An endogenous plasmid of *Brevibacterium flavum* MJ-233 strain (FERM BP-1497) was cured by a conventional method (Wolf H et al., J. Bacteriol. 1983, 156(3) 1165-1170; Kurusu Y et al., Agric Biol Chem. 1990, 54(2) 443-7), and the obtained plasmid-cured strain *Brevibacterium flavum* MJ233-ES was used for the following transformation.
The *Brevibacterium flavum* MJ-233 strain was cultured until the late stage of logarithmic growth phase in a 10mL A medium (2g of urea, 7 g of $(NH_4)_2SO_4$, 0.5 g of $KH_2PO_4$, 0.5 g of $K_2HPO_4$, 0.5 g of $MgSO_4 \cdot 7H_2O$, 6 mg of $FeSO_4 \cdot 7H_2O$, 6 mg of $MnSO_4 \cdot 4\text{-}5H_2O$, 200 μg of biotin, 100 μg of thiamine, 1 g of yeast extract, 1 g of casamino aid, and 20 g of glucose dissolved in 1 L of distilled water). The obtained bacterial cells were used to prepare a genomic DNA by the method described in the above section (A) of Reference Example 1.
(B) Cloning of a lactate dehydrogenase gene
A lactate dehydrogenase gene of MJ233 strain was obtained by performing PCR by: using the DNA prepared in the above section (A) as a template; and using synthetic DNAs (SEQ ID NOS: 5 and 6) designed based on the nucleotide sequence of the gene described in JP11-206385A. The composition of the reaction solution is as follows: 1 μL of the template DNA, 0.2 μL of TaqDNA polymerase (available from Takara Shuzo Co., Ltd.), 1 time concentration of a supplied buffer, 0.2 μM of respective primers, and 0.25 μM dNTPs were mixed, and a total volume of the reaction liquid was adjusted to 20 μL.
Reaction temperature condition is as follows: The DNA Thermal Cycler PTC-2000 manufactured by MJ Research Co., Ltd. was used and a cycle of 94°C for 20 seconds, 55°C for 20 seconds, and 72°C for 1 minute was repeated 30 times. For the first cycle, heat-retention at 94°C was conducted for 1 minute 20 seconds. For the last cycle, the heat-retention at 72°C was conducted for 5 minutes.
The amplified product was analyzed by separating in 0.75% agarose (SeaKem GTG agarose, available from FMC BioProducts) gel electrophoresis and visualizing with ethidium bromide staining, to thereby detect a fragment of about 0.95 kb. The target DNA fragment was recovered from the gel by using QIAQuick Gel Extraction Kit (available from QIAGEN).
The recovered DNA fragment was mixed with the PCR product-cloning vector pGEM-T Easy (available from Promega Corporation) and ligated thereto using Ligation Kit ver. 2 (available from Takara Shuzo Co., Ltd.), and the obtained plasmid DNA was used to transform *Escherichia coli* (DH5α strain). The obtained recombinant *Escherichia coli* was spread on an LB agar medium containing 50 μg/mL ampicillin and 50 μg/mL X-Gal.
Clones each forming a white colony on this medium were subjected to liquid culture by a conventional method, and then the plasmid DNA was purified. The obtained plasmid DNA was cleaved with restriction enzymes SacI and SphI. The plasmid DNA was confirmed to have an insert of about 1.0 kb and named pGEMT/CgLDH.

(C) Construction of a plasmid for disrupting lactate dehydrogenase gene
pGEMT/CgLDH prepared in the above section (B) was digested with restriction enzymes EcoRV and XbaI to remove a coding region of lactate dehydrogenase of about 0.25 kb. The each end of the remaining DNA fragment of about 3.7 kb was made blunt by the Klenow Fragment and self-ligated by using the Ligation Kit ver. 2 (available from Takara Shuzo Co., Ltd.), and the obtained plasmid was used to transform the *Escherichia coli* (DH5α strain). The obtained recombinant *Escherichia coli* was spread on an LB agar medium containing 50 μg/mL ampicillin.
A strain grown on this medium was subjected to liquid culture by a conventional method, and then the plasmid DNA was isolated. The obtained plasmid DNA was digested with restriction enzymes SacI and SphI. A clone having an insert of about 0.75 kb was selected and named pGEMT/ΔLDH.
Next, the DNA fragment of about 0.75 kb obtained by digesting pGEMT/ΔLDH with the restriction enzymes SacI and SphI was separated in 0.75% agarose gel electrophoresis and recovered, to prepare a lactate dehydrogenase gene fragment in which a part of its region is deleted. This DNA fragment was mixed with the pKMB1 constructed in Reference Example 1 digested with the restriction enzymes SacI and SphI, and ligated thereto by using the Ligation Kit ver. 2 (available from Takara Shuzo Co., Ltd.), and the obtained plasmid DNA was used to transform the *Escherichia coli* (DH5α strain). The obtained recombinant *Escherichia coli* was spread on an LB agar medium containing 50 μg/mL kanamycin and 50 μg/mL X-Gal.
Clones each forming a white colony on this medium was subjected to liquid culture by a conventional method, and then the plasmid DNA was isolated. The obtained plasmid DNA was digested with restriction enzymes SacI and SphI. A clone having an insert of about 0.75 kb was selected and named pKMB1/ΔLDH (Fig. 2).

(D) Construction of lactate dehydrogenase gene-disrupted strain derived from *Brevibacterium flavum* MJ233-ES strain
A plasmid DNA to be used for transformation of the *Brevibacterium flavum* MJ-233 strain was isolated from *Escherichia coli* JM110 strain transformed with pKMB1/ΔLDH by a calcium chloride method (Journal of Molecular Biology, 53, 159, 1970).
The transformation of the *Brevibacterium flavum* MJ233-ES strain was performed by an electric pulse method (Res. Microbiol., Vol.144, p.181-185, 1993), and the obtained transformant was spread on an LBG agar medium (10 g of tryptone, 5 g of yeast extract, 5 g of NaCl, 20 g of glucose, and 15 g of agar dissolved in 1 L of distilled water) containing 50 μg/mL kanamycin.
Because pKMB1/ΔLDH is a plasmid incapable of replicating in the *Brevibacterium flavum* MJ233-ES strain, a strain grown on this medium must have a kanamycin-resistant gene and SacB gene derived from the plasmid on its genome, as a result of homologous recombination between a lactate dehydrogenase gene on the plasmid and the same gene on the genome of the *Brevibacterium flavum* MJ-233 strain.
Next, the strain obtained by homologous recombination was subjected to liquid culture on an LBG medium containing 50 μg/mL kanamycin. The culture solution supposed to contain about 1,000,000 bacterial cells was spread on an LBG medium containing 10% sucrose. As a result, about 10 sucrose-insensitive strains in which the SacB gene was removed by the second homologous recombination were obtained.
The obtained strains include: a strain in which the lactate dehydrogenase gene was replaced by a deletion type derived from pKMB1/11LDH; and a strain in which the lactate dehydrogenase gene reverted to a wild type. Whether the lactate dehydrogenase gene is a deletion type or a wild type can be confirmed easily by subjecting a bacterial strain obtained by liquid culture in an LBG medium to direct PCR and detecting the lactate dehydrogenase gene. Analysis of the lactate dehydrogenase gene by using primers (SEQ ID NOS: 7 and 8) for PCR amplification results in a DNA fragment of 720 bp for a wild type and a DNA fragment of 471 bp for a deletion type.
As a result of the analysis of the sucrose-insensitive strain by the above-mentioned method, a strain having only a deletion type gene was selected and named *Brevibacterium flavum* MJ233/ΔLDH.

Reference Example 3. Construction of expression vector for coryneform bacterium

(A) Preparation of a promoter fragment for coryneform bacterium

A DNA fragment (hereinafter, referred to TZ4 promoter) shown in SEQ ID NO: 4 in JP07-95891A and reported to have high promoter activity in a coryneform bacterium was used. The promoter fragment was obtained by performing PCR by using the *Brevibacterium flavum* MJ233 genomic DNA prepared in the section (A) of Reference Example 2 as a template; and using synthetic DNAs (SEQ ID NOS: 9 and 10) designed based on a sequence described as SEQ ID NO: 4 in JP07-95891A, as primers.
The composition of the reaction solution is as follows: 1 μL of the template DNA, 0.2 μL of PfxDNA polymerase (available from Invitrogen Japan K.K.), 1 time concentration of a supplied buffer, 0.3 μM of respective primers, 1 mM $MgSO_4$, and 0.25 μM dNTPs were mixed, and a total volume of the reaction solution was adjusted to 20 μL.
Reaction temperature condition is as follows: The DNA Thermal Cycler PTC-2000 manufactured by MJ Research Co., Ltd. was used and a cycle of 94°C for 20 seconds, 60°C for 20 seconds, and 72°C for 30 seconds was repeated 35 times. For the first cycle, heat-retention at 94°C was conducted for 1 minute 20 seconds. For the last cycle, the heat-retention at 72°C was conducted for 2 minutes.
The amplified product was analyzed by separating in 2.0% agarose (SeaKem GTG agarose, available from FMC Bio-Products) gel electrophoresis and visualizing with ethidium bromide staining, to thereby detect a fragment of about 0.25 kb. The target DNA fragment was recovered from the gel by using the QIAQuick Gel Extraction Kit (available from QIAGEN).
The 5'-end of the recovered DNA fragment was phosphorylated with T4 Polynucleotide Kinase (available from Takara Shuzo Co., Ltd.) and was ligated to an SmaI site of an *Escherichia coli* vector pUC19 (Takara Shuzo Co., Ltd.) by using the Ligation Kit ver. 2 (available from Takara Shuzo Co., Ltd.), and the obtained plasmid DNA was used to transform the *Escherichia coli* (DH5α strain). The obtained recombinant *Escherichia coli* was spread on an LB agar medium containing 50 μg/mL ampicillin and 50 μg/mL X-Gal.
Six clones each forming a white colony on this medium were subjected to liquid culture by a conventional method, and then the plasmid DNA was isolated, and the nucleotide sequence was determined. Of those, a clone having a TZ4 promoter inserted therein so to have transcription activity in an opposite direction with respect to the lac promoter on pUC 19 was selected and named pUC/TZ4.
Next, a DNA linker consisting of synthetic DNAs (SEQ ID NOS: 11 and 12) each having phosphorylated 5'-ends and having sticky ends corresponding to each of BamHI and PstI was added to the DNA fragment prepared by digesting pUC/TZ4 with restriction enzymes BamHI and PstI, and ligated with each other by using the Ligation Kit ver. 2 (available from Takara Shuzo Co., Ltd.), and the obtained plasmid DNA was used to transform the *Escherichia coli* (DH5α strain). This DNA linker includes a ribosome binding sequence (AGGAGG) and a cloning site (the order of PacI, NotI, and ApaI from upstream) arranged downstream of the ribosome binding sequence.
Clones each forming a white colony on this medium were subjected to liquid culture by a conventional method, and then the plasmid DNA was isolated. Of the obtained plasmid DNAs, a plasmid DNA capable of being cleaved with a restriction enzyme NotI was selected and named pUC/TZ4-SD.
A promoter fragment of about 0.3 kb was obtained by digesting the pUC/TZ4-SD with a restriction enzyme PstI, making its end blunt with the Klenow Fragment, and cleaving the resultant DNA with a restriction enzyme KpnI, and separated in 2.0% agarose gel electrophoresis, and recovered.
(B) Construction of expression vector for coryneform bacterium
pHSG298par-rep described in JP12-93183A was used as a plasmid capable of stable and autonomous replication in coryneform bacteria. This plasmid includes a replicating region and a region having a stabilization function of a natural plasmid pBY503 from *Brevibacterium stationis* IFO12144 strain, a kanamycin resistant gene derived from an *Escherichia coli* vector pHSG298 (Takara Shuzo Co., Ltd.), and a replicating region for *Escherichia coli*.
A DNA was prepared by digesting pHSG298par-rep with a restriction enzyme SseI, making its end blunt with the Klenow Fragment, and digesting the resultant DNA with the restriction enzyme KpnI, and the DNA was mixed with the TZ4 promoter fragment prepared in the above section (A) and ligated thereto by using the Ligation Kit ver. 2 (available from Takara Shuzo Co., Ltd.), and the the obtained plasmid DNA was used to transform the *Escherichia coli* (DH5α strain). The obtained recombinant *Escherichia coli* was spread on an LB agar medium containing 50 μg/mL kanamycin.
A strain grown on this medium was subjected to liquid culture by a conventional method, and then the plasmid DNA was purified. Of the obtained plasmid DNA, a plasmid DNA capable of being digested with the restriction enzyme NotI was selected and named pTZ4 (Fig. 3 shows the construction procedure).

Reference Example 4. Construction of pyruvate carboxylase activity-enhanced strain

(A) Acquisition of a pyruvate carboxylase gene

A pyruvate carboxylase gene derived from the *Brevibacterium flavum* MJ233 strain was obtained by performing PCR by using the DNA prepared in the section (A) of Reference Example 2 as a template; and using synthetic DNAs (SEQ ID NOS: 13 and 14) designed based on a sequence of a pyruvate carboxylase gene of a *Corynebacterium glutamicum* ATCC13032 strain whose entire genomic sequence was reported (GenBank Database Accession No. AP005276).
The composition of the reaction solution is as follows: 1 μL of the template DNA, 0.2 μL of PfxDNA polymerase (available from Invitrogen Japan K.K.), 1-fold concentration of the supplied buffer, 0.3 μM of respective primers, 1 mM $MgSO_4$, and 0.25 μM dNTPs were mixed, and a total volume of the reaction liquid was adjusted to 20 μL.
Reaction temperature condition is as follows: The DNA Thermal Cycler PTC-2000 manufactured by MJ Research Co., Ltd. was used and a cycle of 94°C for 20 seconds and 68°C for 4 minutes was repeated 35 times. For the first cycle, heat-retention at 94°C was conducted for 1 minute 20 seconds. For the last cycle, the heat-retention at 68°C was conducted for 10 minutes. After completion of PCR, 0.1 M of Takara Ex Taq (Takara Shuzo Co., Ltd.) was added and kept at 72°C for 30 minutes.
The amplified product was analyzed by separating in 0.75% agarose (SeaKem GTG agarose, available from FMC BioProducts) gel electrophoresis and visualizing with ethidium bromide staining, to thereby detect a fragment of about 3.7 kb. The target DNA fragment was recovered from the gel by using the QIAQuick Gel Extraction Kit (available from QIAGEN).
The recovered DNA fragment was mixed with the PCR product-cloning vector pGEM-TEasy (available from Promega Corporation) and ligated thereto by using the Ligation Kit ver. 2 (available from Takara Shuzo Co., Ltd.), and the obtained plasmid DNA was used to transform *Escherichia coli* (DH5α strain). The obtained recombinant *Escherichia coli* was spread on an LB agar medium containing 50 μg/mL ampicillin and 50 μg/mL X-Gal.
Clones each forming a white colony on this medium were subjected to liquid culture by a conventional method, and then the plasmid DNA was isolated. The obtained plasmid DNA was digested with restriction enzymes PacI and ApaI. The plasmid DNA was confirmed to have an insert of about 3.7 kb and named pGEM/MJPC.
A nucleotide sequence of the insert in pGEM/MJPC was determined by using the nucleotide sequencing device (model 377 XL, manufactured by Applied Biosystems) and BigDye Terminator Cycle Sequencing Kit ver. 3 (manufactured by Applied Biosystems). SEQ ID NO: 15 shows the determined nucleotide sequence and a predicted amino acid sequence. The amino acid sequence is extremely highly homologous (99.4%) to that derived from the *Corynebacterium glutamicum* ATCC13032 strain, concluding that the pGEM/MJPC insert was a pyruvate carboxylase gene derived from the *Brevibacterium flavum* MJ233 strain.

(B) Construction of plasmid for enhancing pyruvate carboxylase activity
Next, the pyruvate carboxylase gene fragment of about 3.7kb obtained by digesting pGEM/MJPC with the restriction enzymes PacI and ApaI in the above section (A) was separated in 0.75% agarose gel electrophoresis, and recovered. This DNA fragment was mixed with pTZ4 digested with the restriction enzymes PacI and ApaI in Reference Example 3 and ligated thereto by using the Ligation Kit ver. 2 (available from Takara Shuzo Co., Ltd.), and the obtained plasmid DNA was used to transform the *Escherichia coli* (DH5α strain). The obtained recombinant *Escherichia coli* was spread on an LB agar medium containing 50 μg/mL kanamycin.
Strains grown on this medium were subjected to liquid culture by a conventional method, and then the plasmid DNA was purified. The obtained plasmid DNA was digested with restriction enzymes PacI and ApaI. A clone having an insert of about 3.7 kb was selected and named pMJPC1 (Fig. 4).

(C) Transformation of *Brevibacterium flavum* MJ233/ΔLDH strain
A plasmid DNA pMJPC1 which is capable of replicating in the *Brevibacterium flavum* MJ233 strain was isolated from the *Escherichia coli* (DH5α strain) transformed in the above section (B).
The transformation of the *Brevibacterium flavum* MJ233/ΔLDH strain was performed by the electric pulse method (Res. Microbiolo., Vol.144, p.181-185, 1993), and the obtained transformant was spread on an LBG agar medium (10 g of tryptone, 5 g of yeast extract, 5 g of NaCl, 20 g of glucose, and 15 g of agar dissolved in 1 L of distilled water) containing 50 μg/mL kanamycin.
A strain grown on this medium was subjected to liquid culture by a conventional method, and then the plasmid DNA was extracted and analyzed with restriction enzyme digestion. The results confirmed that the strain retained pMJPC1, and the strain was named *Brevibacterium flavum* MJ233/PC/ALDH strain.

Reference Example 5. Cloning of *Escherichia coli* fumarate reductase gene

(A) Extraction of *Escherichia coli* DNA

*Escherichia coli* JM109 strain was incubated in 10 ml of LB culture medium until the late stage of the logarithmic growth phase, and the resulting bacterial cells were then subjected to the method described in the section (A) of Reference Example 1 to prepare a genomic DNA.

(B) Cloning of *Escherichia coli* fumarate reductase gene

The *Escherichia coli* fumarate reductase gene was obtained by PCR using the DNA prepared in the above section (A) as a template and synthetic DNAs (SEQ ID NOS: 16 and 17) designed on the basis of the sequence of the gene of *Escherichia coli* K12-MG1655 strain whose the whole genome sequence had been reported (GenBank Database Accession NO. U00096).

Composition of reaction solution is as follows: 1 μL of template DNA, 0.2 μL of PfxDNA polymerase (manufactured by Invitrogen Co., Ltd.), 1-fold concentration of the supplied buffer, 0.3 μM of respective primers, 1 mM $MgSO_4$, and 0.25 μM of dNTPs were mixed, and the total volume was adjusted to 20 μL.

Reaction temperature condition is as follows: The DNA Thermal Cycler PTC-2000 manufactured by MJ Research Co., Ltd. was used and a cycle of 94°C for 20 seconds and 68°C for 4 minutes was repeated 35 times. For the first cycle, heat-retention at 94°C was conducted for 1 minute 20 seconds. For the last cycle, the heat-retention at 68°C was conducted for 10 minutes. After completion of PCR, 0.1 μL of Takara Ex Taq (Takara Shuzo Co., Ltd.) was added and kept at 72°C for 30 minutes.

The amplified product was analyzed by separating in 0.75% agarose (Sea Kem GTG agarose: manufactured by FMC BioProducts) gel electrophoresis and then visualized with ethidium bromide staining, thereby detecting a fragment of about 3.8 kb. The DNA fragment of interest was isolated from the gel by means of QIA Quick Gel Extraction Kit (manufactured by QIAGEN).

The recovered DNA fragment was mixed with the PCR product-cloning vector pT7 Blue T-Vector (manufactured by Novagen) and ligated thereto by Ligation Kit ver. 2 (manufactured by Takara Shuzo Co., Ltd.), and the obtained plasmid DNA was used to transform *Escherichia coli* (DH5α strain). The obtained recombinant *Escherichia coli* was spread on an LB agar culture medium containing 50 μg/mL ampicillin and 50 μg/mL X-Gal.

A clone forming a white colony on the culture medium was incubated in liquid culture according to a conventional method, followed by purifying the plasmid DNA. The resulting plasmid DNA was digested with restriction enzymes HindIII and KpnI, thereby confirming an insert fragment of about 3.9 kb, and named pFRD6.0.

The nucleotide sequence of the insert fragment of pFRD6.0 was determined using the nucleotide sequencing device (model 377XL) manufactured by Applied Biosystems, Inc. and BigDye Terminator Cycle Sequencing Kit ver. 3. The resulting nucleotide sequence is described in SEQ ID NO: 18.

Reference Example 6. Construction of a strain with enhanced activities of pyruvate carboxylase/fumarate reductase

(A) Modification of a restriction enzyme recognition site of pMJPC1

pMJPC1 constructed in Reference Example 3 was completely digested with the restriction enzyme KpnI, and its 5'-ends was dephosphorylated by a reaction with Calf intestine Alkaline Phosphatase (Takara Shuzo Co., Ltd.). The DNA linker consisting of the synthetic DNAs with phosphorylated 5'-ends (SEQ ID NOS: 19 and 20) was mixed with the obtained fragment and ligated thereto using the Ligation Kit ver. 2 (available from Takara Shuzo Co., Ltd.), and the obtained plasmid DNA was used to transform the *Escherichia coli* (DH5α strain). The obtained recombinant *Escherichia coli* was spread on an LB agar medium containing 50 μg/mL kanamycin.

A strain grown on this medium was subjected to liquid culture by a conventional method, and then the plasmid DNA was isolated. Of the obtained plasmid DNA, a plasmid DNA which can be digested with the restriction enzyme NdeI was selected and named pMJPC1.1.

(B) Construction of a plasmid for enhancing activities of pyruvate carboxylase and fumarate reductase

A DNA fragment of about 3.9 kb was obtained by digesting pFRD6.0 prepared in Reference Example 5 with the restriction enzyme HindIII, and making its end blunt with the Klenow Fragment, and digesting with the restriction enzyme KpnI. The DNA fragment was separated in 0.75% agarose gel electrophoresis, and recovered. The prepared fragment containing the *Escherichia coli* fumarate reductase gene was mixed and ligated, by using the Ligation Kit ver. 2 (available from Takara Shuzo Co., Ltd.), to the DNA which was obtained by digesting pMJPC1.1 prepared in the above section (A) with the restriction enzyme NdeI, making its end blunt with the Klenow Fragment, followed by digestion with the restriction enzyme KpnI. The obtained plasmid DNA was used to transform *Escherichia coli* (DH5α strain). The obtained recombinant *Escherichia coli* was spread on an LB agar medium containing 50 μg/mL kanamycin.

A strain grown on this medium was subjected to liquid culture by a conventional method, and then the plasmid DNA was isolated. The obtained plasmid DNA showed fragments of 505, 2,132, 2,675, 3,775, and 4,193 bp after restriction enzyme HindIII digestion. Thus, it was concluded that the DNA has the structure shown in Fig. 5, and the plasmid was named pFRPC 1.1.

(B) Transformation of *Brevibacterium flavum* MJ233/ΔLDH strain

The transformation of the *Brevibacterium flavum* MJ233/ΔLDH strain with pFRPC1.1 was performed by the method described in the section (C) of Reference Example 4, to thereby obtain a strain having the plasmid pFRPC1.1. This strain was named *Brevibacterium flavum* MJ233/FRD/PC/ALDH strain.

INDUSTRIAL APPLICABILITY

The present invention provides a novel method for producing organic acid ammonium solution. The method of the present invention allows efficient production of organic acid ammonium solution such as ammonium succinate solution by recycling carbonic acid, ammonia, and the like to be used as a neutralizer or in salt-exchange. Succinic acid to be obtained from the ammonium succinate solution which is produced by the present invention is useful as a raw material for polymers such as biodegradable polyester and polyamide, foods, pharmaceuticals, cosmetics, and the like.

**Claims**

1. A method for producing solution of organic acid ammonium, which comprises a fermentation step of obtaining fermentation broth containing organic acid magnesium by using a microorganism having an organic acid-producing ability in the presence of a magnesium compound, a salt-exchanging step of subjecting the organic acid magnesium contained in said fermentation broth using an ammonium compound to produce organic acid ammonium and a magnesium compound, and a magnesium-separating step of separating the produced magnesium compound to obtain solution of organic acid ammonium.

2. The method for producing solution of organic acid ammonium according to claim 1, wherein the magnesium compound separated in said magnesium-separating step is recycled as the magnesium compound in said fermentation step.

3. The method for producing solution of organic acid ammonium according to claim 1 or 2, wherein the magnesium compound is magnesium carbonate and the ammonium compound is ammonium carbonate.

4. The method for producing solution of organic acid ammonium according to any one of claims 1 to 3, wherein ammonium carbonate generated by providing carbon dioxide and ammonia into the fermentation broth is used as the ammonium compound.

5. The method for producing solution of organic acid ammonium according to claim 4, wherein carbon dioxide is provided in a molar amount 0.3 to 10 times larger than that of magnesium in the fermentation broth.

6. The method for producing solution of organic acid ammonium according to claim 4, further comprising the steps of heating the solution of organic acid ammonium obtained in said magnesium-separating step to vaporize and separate excess carbon dioxide and ammonia existing in the solution, and recycling the separated carbon dioxide and ammonia in said salt-exchanging step.

7. The method for producing solution of organic acid ammonium according to claim 1, further comprising the steps of heating the magnesium carbonate separated in said magnesium-separating step to be decomposed into magnesium oxide and carbon dioxide, generating magnesium hydroxide by adding water into the magnesium oxide, and recycling the magnesium hydroxide as the magnesium compound in said fermentation step.

8. The method for producing solution of organic acid ammonium according to claim 1, wherein said magnesium compound is magnesium hydroxide or a mixture of magnesium hydroxide and magnesium carbonate, and ammonia is used in said salt-exchanging step.

9. The method for producing solution of organic acid ammonium according to claim 8, further comprising the steps of adding ammonium carbonate into the solution of organic acid ammonium obtained in said magnesium-separating step to further generate organic acid ammonium and magnesium carbonate, and obtaining solution of organic acid ammonium by separating the magnesium carbonate.

10. The method for producing solution of organic acid ammonium according to any one of claims 1 to 9, wherein said salt-exchanging step is performed at the pH range between 7 and 12.

11. The method for producing solution of organic acid ammonium according to any one of claims 1 to 10, wherein said organic acid is succinic acid and said organic acid ammonium is ammonium succinate.

12. The method for producing solution of organic acid ammonium according to claim 1, further comprising the steps of heating or drying a double salt of magnesium carbonate and ammonium carbonate contained in the magnesium compound separated by said magnesium-separating step to remove ammonium carbonate and obtain magnesium carbonate, and recycling the magnesium carbonate in said fermentation step.

13. The method for producing solution of organic acid ammonium according to claim 12, wherein ammonium carbonate is removed so that molar content of ammonia in said double salt becomes one tenth or lower with respect to that of magnesium.

14. The method for producing solution of organic acid ammonium according to claim 12, wherein ammonium carbonate is removed so that molar content of ammonia in said double salt becomes one 30th or lower with respect to that of magnesium.

**15.** The method for producing solution of organic acid ammonium according to claim 12, wherein said double salt is heated at the temperature of not less than 160°C.

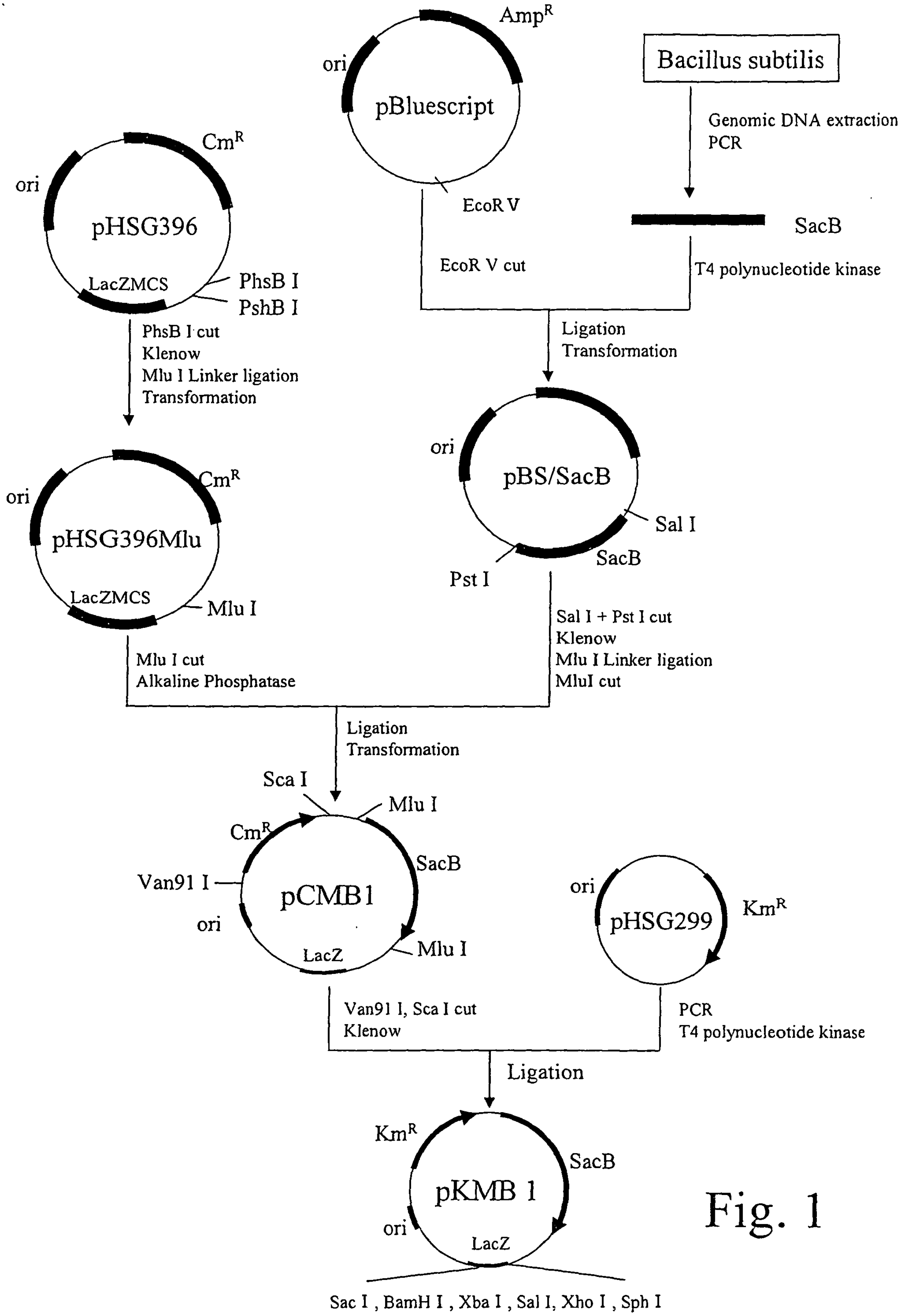
AmpR
ori
pBluescript
Bacillus subtilis
Genomic DNA extraction
PCR
EcoR V
SacB
EcoR V cut
T4 polynucleotide kinase
CmR
ori
pHSG396
LacZMCS
PhsB I
PshB I
PhsB I cut
Klenow
Mlu I Linker ligation
Transformation
Ligation
Transformation
ori
pBS/SacB
Sal I
SacB
Pst I
CmR
ori
pHSG396Mlu
LacZMCS
Mlu I
Sal I + Pst I cut
Klenow
Mlu I Linker ligation
MluI cut
Mlu I cut
Alkaline Phosphatase
Ligation
Transformation
Sca I
Mlu I
CmR
SacB
Van91 I
pCMB1
ori
LacZ
Mlu I
ori
pHSG299
KmR
Van91 I, Sca I cut
Klenow
PCR
T4 polynucleotide kinase
Ligation
KmR
SacB
pKMB 1
ori
LacZ
Sac I , BamH I , Xba I , Sal I, Xho I , Sph I

Fig. 1

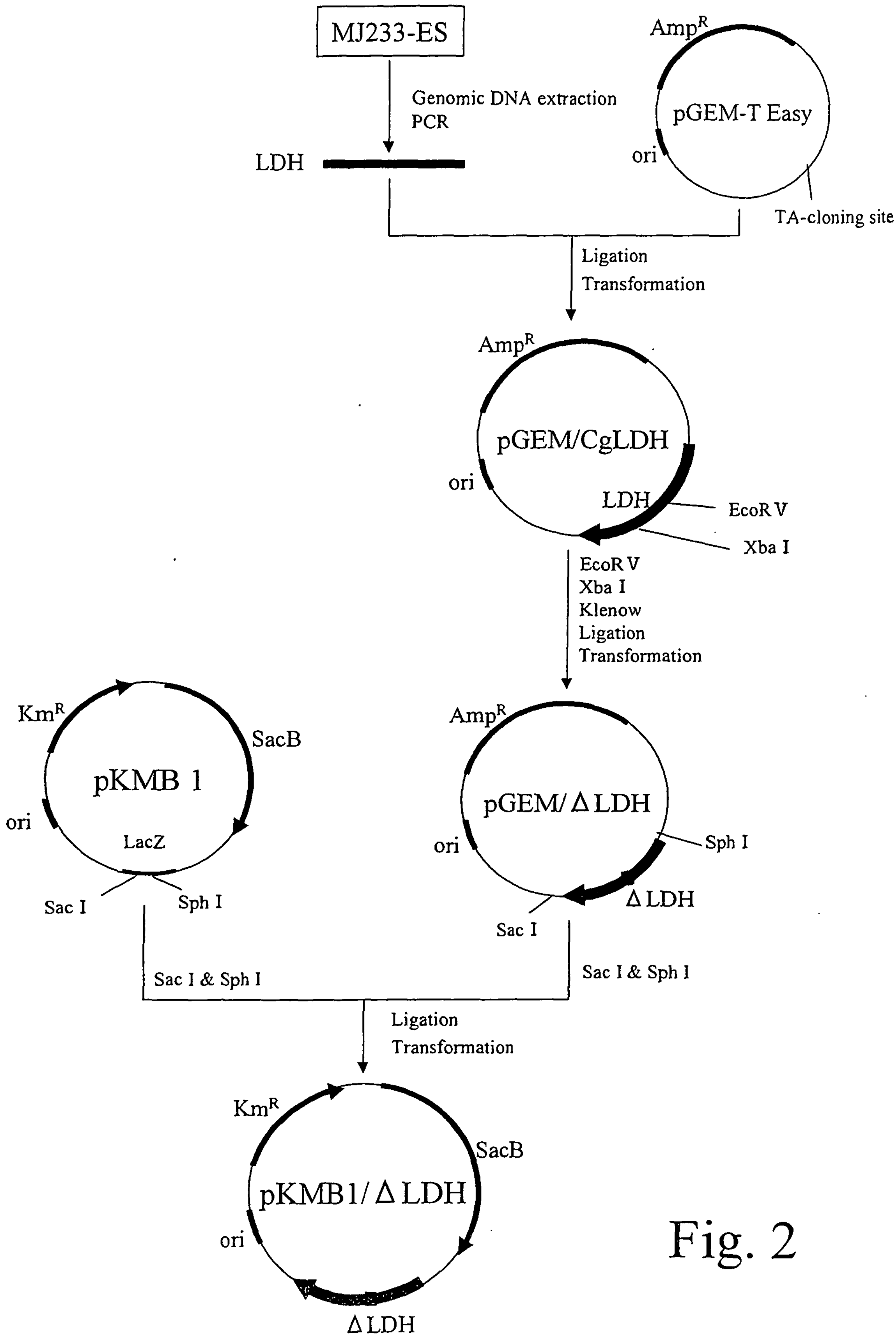
MJ233-ES
Genomic DNA extraction
PCR
LDH
AmpR
pGEM-T Easy
ori
TA-cloning site
Ligation
Transformation
AmpR
pGEM/CgLDH
ori
LDH
EcoR V
Xba I
EcoR V
Xba I
Klenow
Ligation
Transformation
KmR
SacB
pKMB 1
ori
LacZ
Sac I
Sph I
AmpR
pGEM/Δ LDH
ori
Sph I
Δ LDH
Sac I
Sac I & Sph I
Sac I & Sph I
Ligation
Transformation
KmR
SacB
pKMB1/Δ LDH
ori
Δ LDH

Fig. 2

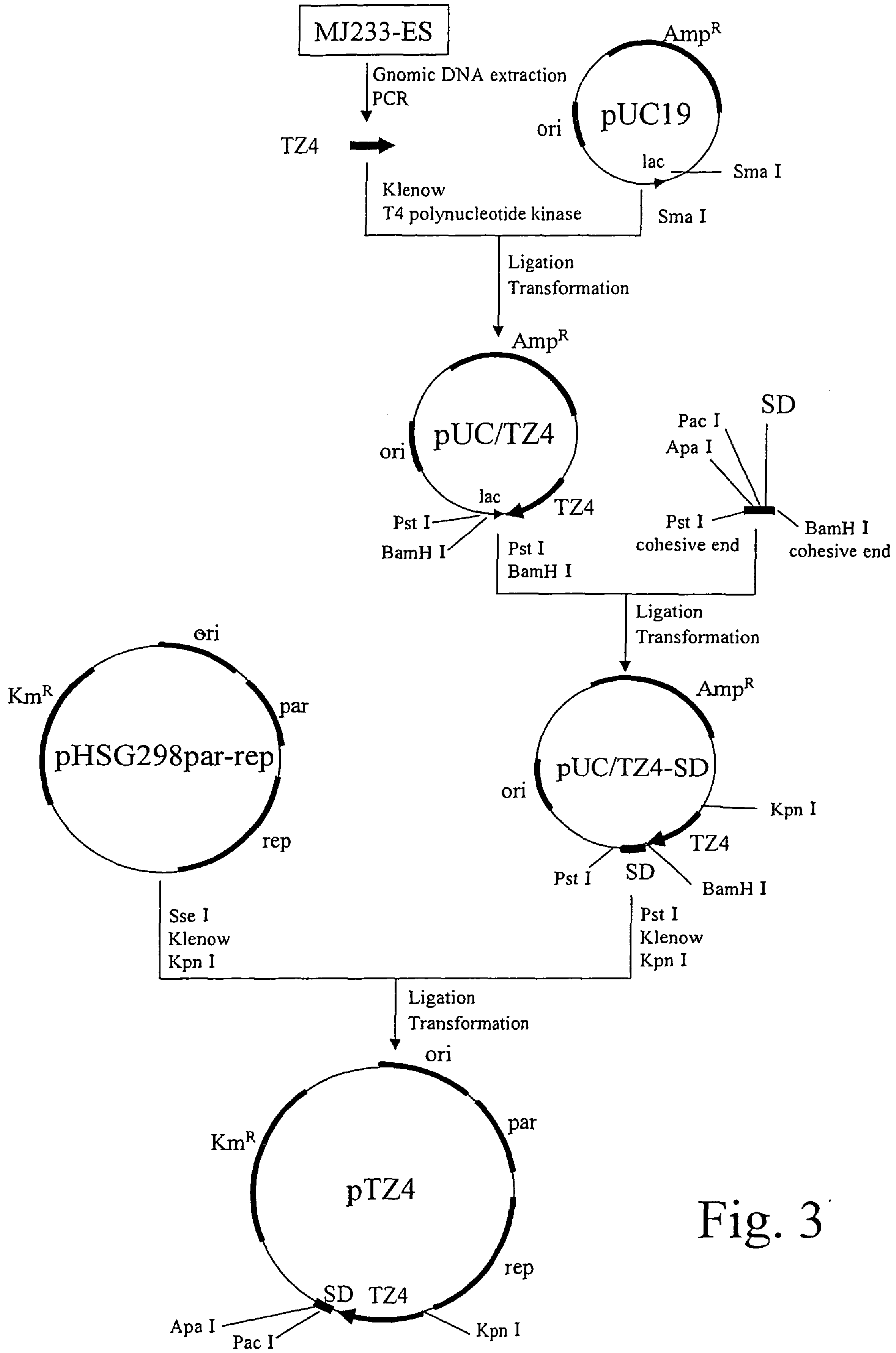
MJ233-ES
Gnomic DNA extraction
PCR
TZ4
AmpR
pUC19
ori
lac
Sma I
Klenow
T4 polynucleotide kinase
Sma I
Ligation
Transformation
AmpR
pUC/TZ4
ori
lac
TZ4
Pst I
BamH I
SD
Pac I
Apa I
Pst I
cohesive end
BamH I
cohesive end
Pst I
BamH I
Ligation
Transformation
ori
KmR
par
pHSG298par-rep
rep
AmpR
pUC/TZ4-SD
ori
Kpn I
TZ4
Pst I
SD
BamH I
Sse I
Klenow
Kpn I
Pst I
Klenow
Kpn I
Ligation
Transformation
ori
par
KmR
pTZ4
rep
SD
TZ4
Apa I
Pac I
Kpn I

Fig. 3

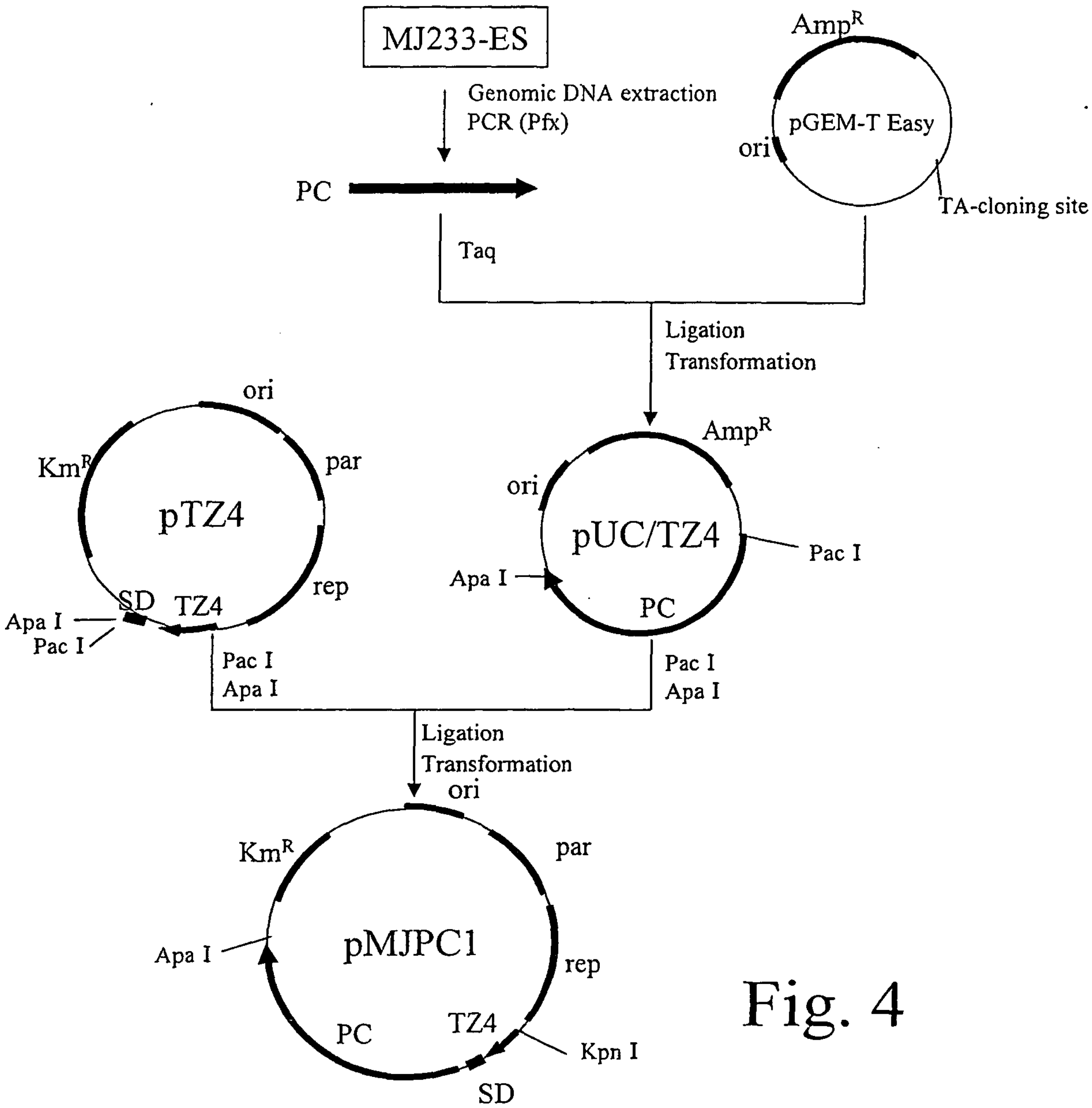
MJ233-ES
Genomic DNA extraction
PCR (Pfx)
PC
Taq
AmpR
pGEM-T Easy
ori
TA-cloning site
Ligation
Transformation
ori
par
KmR
pTZ4
rep
SD
TZ4
Apa I
Pac I
Pac I
Apa I
AmpR
ori
pUC/TZ4
Pac I
Apa I
PC
Pac I
Apa I
Ligation
Transformation
ori
KmR
par
pMJPC1
Apa I
rep
PC
TZ4
Kpn I
SD

Fig. 4

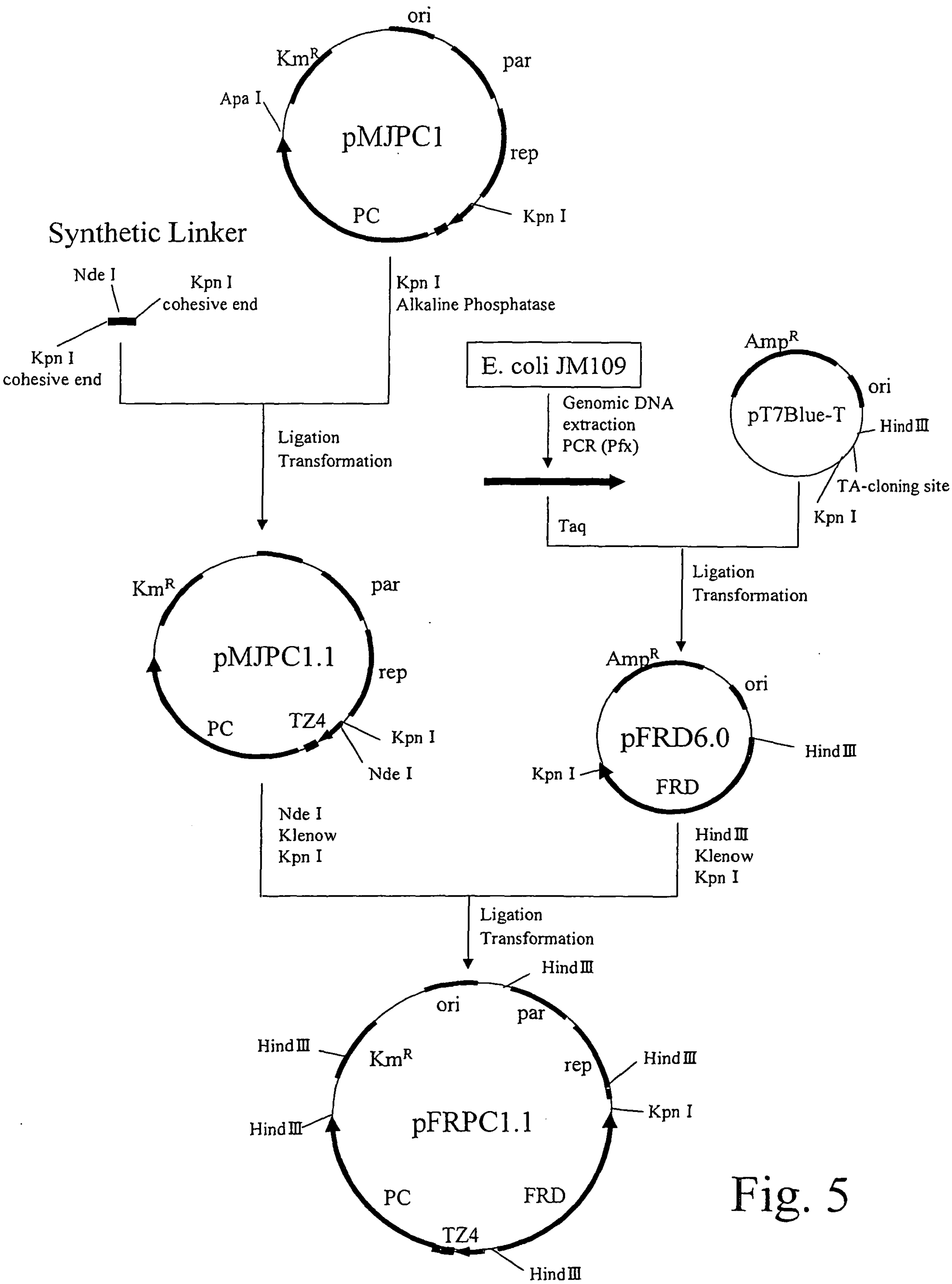
ori
Km^R
par
Apa I
pMJPC1
rep
PC
Kpn I
Synthetic Linker
Nde I
Kpn I
cohesive end
Kpn I
Alkaline Phosphatase
Kpn I
cohesive end
E. coli JM109
Amp^R
ori
pT7Blue-T
Hind III
Genomic DNA
extraction
PCR (Pfx)
TA-cloning site
Kpn I
Taq
Ligation
Transformation
Ligation
Transformation
Km^R
par
pMJPC1.1
rep
PC
TZ4
Kpn I
Nde I
Amp^R
ori
pFRD6.0
Hind III
Kpn I
FRD
Nde I
Klenow
Kpn I
Hind III
Klenow
Kpn I
Ligation
Transformation
Hind III
ori
par
Hind III
Km^R
rep
Hind III
Hind III
pFRPC1.1
Kpn I
PC
FRD
TZ4
Hind III

Fig. 5

## INTERNATIONAL SEARCH REPORT

International application No.
PCT/JP2004/016437

A. CLASSIFICATION OF SUBJECT MATTER
Int.Cl[7] C12P7/46

According to International Patent Classification (IPC) or to both national classification and IPC

B. FIELDS SEARCHED

Minimum documentation searched (classification system followed by classification symbols)
Int.Cl[7] C12P7/46

Documentation searched other than minimum documentation to the extent that such documents are included in the fields searched

Electronic data base consulted during the international search (name of data base and, where practicable, search terms used)
CAPLUS/MEDLINE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 7-194387 A (BASF AG.),<br>01 August, 1995 (01.08.95),<br>Claim 1; examples<br>& DE 4341770 A & EP 658628 A1<br>& US 5453365 A | 1-5,10<br>6-9,11-15 |
| Y | JP 2001-514900 A (APPLIED CARBOCHEMICALS),<br>18 September, 2001 (18.09.01),<br>Particularly, Claim 1; Par. Nos. [0009], [0019], [0036] to [0037]<br>& AU 9107598 A & WO 99/09196 A1<br>& CA 2301177 A & ZA 9807428 A<br>& US 5958744 A & BR 9815652 A<br>& EP 1005562 A | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 January, 2005 (26.01.05) | 15 February, 2005 (15.02.05) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No. PCT/JP2004/016437

C (Continuation). DOCUMENTS CONSIDERED TO BE RELEVANT

| Category* | Citation of document, with indication, where appropriate, of the relevant passages | Relevant to claim No. |
|---|---|---|
| Y | WO 1998/33930 A1 (LOCKHEED MARTIN ENERGY RESEARCH CORP.), 06 August, 1998 (06.08.98), Particularly, page 19, lines 28 to 30 & JP 2000-515389 A & EP 1012323 A & AU 7605896 A & CA 2278892 A & US 5770435 A & CN 1202930 A & US 5869301 A & HU 9901992 A & BR 9611220 A & BR 9806822 A & HU 001997 A | 1-15 |
| E,X | JP 2004-196768 A (Mitsubishi Chemical Corp.), 15 July, 2004 (15.07.04), Particularly, Claim 9 (Family: none) | 1-15 |

Form PCT/ISA/210 (continuation of second sheet) (January 2004)

SEQUENCE LISTING

<110> Mitsubishi Chemical Corporation
Ajinomoto Co., Inc.

<120> Method for producing organic acid ammonium solution

<130> A4151-C4255

<150> JP2003-378732
<151> 2003-11-07

<150> JP2004-79488
<151> 2004-03-19

<160> 20

<170> PatentIn version 3.1

<210> 1
<211> 34
<212> DNA
<213> Artificial

<220>
<223> primer

<400> 1
ccttttaac ccatcacata tacctgccgt tcac 34

<210> 2
<211> 32
<212> DNA
<213> Artificial

<220>
<223> primer

<400> 2
aaaggttagg aatacggtta gccatttgcc tg 32

<210> 3
<211> 21
<212> DNA

<213> Artificial

<220>
<223> primer

<400> 3
gaggtctgcc tcgtgaagaa g 21

<210> 4
<211> 27
<212> DNA
<213> Artificial

<220>
<223> primer

<400> 4
ctcattagaa aaactcatcg agcatca 27

<210> 5
<211> 20
<212> DNA
<213> Artificial

<220>
<223> primer

<400> 5
cgatgaaaga aaccgtcggc 20

<210> 6
<211> 20
<212> DNA
<213> Artificial

<220>
<223> primer

<400> 6
cgtcagaaga actgcttctg 20

<210> 7
<211> 23
<212> DNA

<213> Artificial

<220>
<223> primer

<400> 7
agttgcatac gcatacgcac tga 23

<210> 8
<211> 23
<212> DNA
<213> Artificial

<220>
<223> primer

<400> 8
gagactggga ctgcaacgtc ttg 23

<210> 9
<211> 24
<212> DNA
<213> Artificial

<220>
<223> primer

<400> 9
gatctttcag ctgctcacac gtga 24

<210> 10
<211> 27
<212> DNA
<213> Artificial

<220>
<223> primer

<400> 10
gatcttaggt cactaaaact aattcag 27

<210> 11
<211> 39
<212> DNA

<213> Artificial

<220>
<223> primer

<400> 11
gatccaggag gcattaatta agcggccgcg ggccctgca 39

<210> 12
<211> 31
<212> DNA
<213> Artificial

<220>
<223> primer

<400> 12
gggcccgcgg ccgcttaatt aatgcctcct g 31

<210> 13
<211> 43
<212> DNA
<213> Artificial

<220>
<223> primer

<400> 13
accttaatta atgtcgactc acacatcttc aacgcttcca gca 43

<210> 14
<211> 44
<212> DNA
<213> Artificial

<220>
<223> primer

<400> 14
gttgggccca ggtttaggaa acgacgacga tcaagtcgcc acct 44

<210> 15
<211> 3423
<212> DNA

<213> Brevibacterium fluvum

<400> 15

```
atgtcgactc acacatcttc aacgcttcca gcattcaaaa agatcttggt agcaaaccgc      60
ggcgaaatcg cggtccgtgc tttccgtgca gcactcgaaa ccggtgcagc cacggtagct     120
atttaccccc gtgaagatcg gggatcattc caccgctctt ttgcttctga agctgtccgc     180
attggtactg aaggctcacc agtcaaggcg tacctggaca tcgatgaaat tatcggtgca     240
gctaaaaaag ttaaagcaga tgctatttac ccgggatatg gcttcctgtc tgaaaatgcc     300
cagcttgccc gcgagtgcgc ggaaaacggc attacttta ttggcccaac cccagaggtt      360
cttgatctca ccggtgataa gtctcgtgcg gtaaccgccg cgaagaaggc tggtctgcca     420
gttttggcgg aatccacccc gagcaaaaac atcgatgaca tcgttaaaag cgctgaaggc     480
cagacttacc ccatctttgt aaaggcagtt gccggtggtg gcggacgcgg tatgcgcttt     540
gtttcttcac ctgatgagct tcgcaaattg gcaacagaag catctcgtga agctgaagcg     600
gcattcggcg acggttcggt atatgtcgag cgtgctgtga ttaaccccca gcacattgaa     660
gtgcagatcc ttggcgatcg cactggagaa gttgtacacc tttatgaacg tgactgctca     720
ctgcagcgtc gtcaccaaaa agttgtcgaa attgcgccag cacagcattt ggatccagaa     780
ctgcgtgatc gcatttgtgc ggatgcagta aagttctgcc gctccattgg ttaccagggc     840
gcgggaactg tggaattctt ggtcgatgaa aagggcaacc acgttttcat cgaaatgaac     900
ccacgtatcc aggttgagca caccgtgact gaagaagtca ccgaggtgga cctggtgaag     960
gcgcagatgc gcttggctgc tggtgcaacc ttgaaggaat tgggtctgac ccaagataag    1020
atcaagaccc acggtgcggc actgcagtgc cgcatcacca cggaagatcc aaacaacggc    1080
ttccgcccag ataccggaac tatcaccgcg taccgctcac caggcggagc tggcgttcgt    1140
cttgacggtg cagctcagct cggtggcgaa atcaccgcac actttgactc catgctggtg    1200
aaaatgacct gccgtggttc cgattttgaa actgctgttg ctcgtgcaca gcgcgcgttg    1260
gctgagttca ccgtgtctgg tgttgcaacc aacattggtt tcttgcgtgc gttgctgcgt    1320
gaagaggact ttacttccaa gcgcatcgcc accggattta tcggcgatca cccacacctc    1380
cttcaggctc cacctgcgga tgatgagcag ggacgcatcc tggattactt ggcagatgtc    1440
accgtgaaca agcctcatgg tgtgcgtcca aaggatgttg cagcaccaat cgataagctg    1500
cccaacatca aggatctgcc actgccacgc ggttcccgtg accgcctgaa gcagcttgga    1560
ccagcagcgt ttgcccgcga tctccgtgag caggacgcac tggcagttac tgataccacc    1620
ttccgcgatg cacaccagtc tttgcttgcg acccgagtcc gctcattcgc actgaagcct    1680
gcggcagagg ccgtcgcaaa gctgactcct gagcttttgt ccgtggaggc ctggggcggt    1740
gcgacctacg atgtggcgat gcgtttcctc tttgaggatc cgtgggacag gctcgacgag    1800
ctgcgcgagg cgatgccgaa tgtgaacatt cagatgctgc ttcgcggccg caacaccgtg    1860
ggatacaccc catacccaga ctccgtctgt cgcgcgtttg ttaaggaagc tgccacctcc    1920
ggcgtggaca tcttccgcat cttcgacgcg cttaacgacg tctcccagat gcgtccagca    1980
atcgacgcag tcctggagac caacaccgcg gtcgctgaag tggctatggc ttattctggt    2040
gatctttccg atccgaatga aaagctctac accctggatt actacctgaa gatggcagag    2100
gagatcgtca agtctggcgc tcacattctg gctattaagg atatggctgg tctgcttcgc    2160
ccagctgcag ccaccaagct ggtcaccgca ctgcgccgtg aatttgatct gccagtgcac    2220
gtgcacaccc acgacactgc gggtggccag ctggcaacct actttgctgc agctcaagct    2280
ggtgcagatg ctgttgacgg tgcttccgca ccactgtctg gcaccacctc ccagccatcc    2340
ctgtctgcca ttgttgctgc attcgcgcac acccgtcgcg ataccggttt gagcctcgag    2400
gctgtttctg acctcgagcc atactgggaa gcagtgcgcg gactgtacct gccatttgag    2460
tctggaaccc caggcccaac cggtcgcgtc taccgccacg aaatcccagg cggacagctg    2520
tccaacctgc gtgcacaggc caccgcactg ggccttgcgg atcgtttcga actcatcgaa    2580
gacaactacg cggcagttaa tgagatgctg ggacgcccaa ccaaggtcac cccatcctcc    2640
aaggttgttg gcgacctcgc actccacctc gttggtgcgg gtgtggatcc agcagacttt    2700
```

gctgcagatc cacaaaagta cgacatccca gactctgtca tcgcgttcct gcgcggcgag 2760
cttggtaacc ctccaggtgg ctggccagag ccactgcgca cccgcgcact ggaaggccgc 2820
tccgaaggca aggcacctct gacggaagtt cctgaggaag agcaggcgca cctcgacgct 2880
gatgattcca aggaacgtcg caacagcctc aaccgcctgc tgttcccgaa gccaaccgaa 2940
gagttcctcg agcaccgtcg ccgcttcggc aacacctctg cgctggatga tcgtgaattc 3000
ttctacggcc tggtcgaagg ccgcgagact ttgatccgcc tgccagatgt gcgcacccca 3060
ctgcttgttc gcctggatgc gatctccgag ccagacgata agggtatgcg caatgttgtg 3120
gccaacgtca acggccagat ccgcccaatg cgtgtgcgtg accgctccgt tgagtctgtc 3180
accgcaaccg cagaaaaggc agattcctcc aacaagggcc atgttgctgc accattcgct 3240
ggtgttgtca ctgtgactgt tgctgaaggt gatgaggtca aggctggaga tgcagtcgca 3300
atcatcgagg ctatgaagat ggaagcaaca atcactgctt ctgttgacgg caaaatcgat 3360
cgcgttgtgg ttcctgctgc aacgaaggtg gaaggtggcg acttgatcgt cgtcgtttcc 3420
taa 3423

<210> 16
<211> 35
<212> DNA
<213> Artificial

<220>
<223> primer

<400> 16
ccacctgcag gactccacga tcggcaaaga aacga 35

<210> 17
<211> 39
<212> DNA
<213> Artificial

<220>
<223> primer

<400> 17
ggtatttaaa aaggcgcaga gcgtcgtttt gaacatagg 39

<210> 18
<211> 3847
<212> DNA
<213> Escherichia coli

<400> 18
ccacctgcag gactccacga tcggcaaaga aacgacggat ctccgccata atcgccgcgc 60
gttttaataa gttaggaatg gatgcgctcg gctgccagga tgccgtttcg ctcatagtta 120
aatctccagt ttttgacaag ggcacgaagt ctactcgcaa cgcgacggcg agacaaattt 180

tacgcaggaa tcaaacagcg gtgggcagtg actaaaaaaa gcacgatctg atggtttagt 240
aattaaatta atcatcttca gtgataattt agccctcttg cgcactaaaa aaatcgatct 300
cgtcaaattt cagacttatc catcagacta tactgttgta cctataaagg agcagtggaa 360
tagcgttcgc agaccgtaac tttcaggtac ttaccctgaa gtacgtggct gtgggataaa 420
aacaatctgg aggaatgtcg tgcaaacctt tcaagccgat cttgccattg taggcgccgg 480
tggcgcggga ttacgtgctg caattgctgc cgcgcaggca aatccgaatg caaaaatcgc 540
actaatctca aaagtatacc cgatgcgtag ccataccgtt gctgcagaag ggggctccgc 600
cgctgtcgcg caggatcatg acagcttcga atatcacttt cacgatacag tagcgggtgg 660
cgactggttg tgtgagcagg atgtcgtgga ttatttcgtc caccactgcc caaccgaaat 720
gacccaactg gaactgtggg gatgcccatg gagccgtcgc ccggatggta gcgtcaacgt 780
acgtcgcttc ggcggcatga aaatcgagcg cacctggttc gccgccgata agaccggctt 840
ccatatgctg cacacgctgt tccagacctc tctgcaattc ccgcagatcc agcgttttga 900
cgaacatttc gtgctggata ttctggttga tgatggtcat gttcgcggcc tggtagcaat 960
gaacatgatg gaaggcacgc tggtgcagat ccgtgctaac gcggtcgtta tggctactgg 1020
cggtgcgggt cgcgtttatc gttacaacac caacggcggc atcgttaccg gtgacggtat 1080
gggtatggcg ctaagccacg gcgttccgct gcgtgacatg gaattcgttc agtatcaccc 1140
aaccggtctg ccaggttccg gtatcctgat gaccgaaggt tgccgcggtg aaggcggtat 1200
tctggtcaac aaaaatggct accgttatct gcaagattac ggcatgggcc cggaaactcc 1260
gctgggcgag ccgaaaaaca aatatatgga actgggtcca cgcgacaaag tctctcaggc 1320
cttctggcac gaatggcgta aaggcaacac catctccacg ccgcgtggcg atgtggttta 1380
tctcgacttg cgtcacctcg gcgagaaaaa actgcatgaa cgtctgccgt tcatctgcga 1440
actggcgaaa gcgtacgttg gcgtcgatcc ggttaaagaa ccgattccgg tacgtccgac 1500
cgcacactac accatgggcg gtatcgaaac cgatcagaac tgtgaaaccc gcattaaagg 1560
tctgttcgcc gtgggtgaat gttcctctgt tggtctgcac ggtgcaaacc gtctgggttc 1620
taactccctg gcggaactgg tggtcttcgg ccgtctggcc ggtgaacaag cgacagagcg 1680
tgcagcaact gccggtaatg gcaacgaagc ggcaattgaa gcgcaggcag ctggcgttga 1740
acaacgtctg aaagatctgg ttaaccagga tggcggcgaa aactgggcga agatccgcga 1800
cgaaatgggc ctggctatgg aagaaggctg cggtatctac cgtacgccgg aactgatgca 1860
gaaaaccatc gacaagctgg cagagctgca ggaacgcttc aagcgcgtgc gcatcaccga 1920
cacttccagc gtgttcaaca ccgacctgct ctacaccatt gaactgggcc acggtctgaa 1980
cgttgctgaa tgtatggcgc actccgcaat ggcacgtaaa gagtcccgcg gcgcgcacca 2040
gcgtctggac gaaggttgca ccgagcgtga cgacgtcaac ttcctcaaac acaccctcgc 2100
cttccgcgat gctgatggca cgactcgcct ggagtacagc gacgtgaaga ttactacgct 2160
gccgccagct aaacgcgttt acggtggcga agcggatgca gccgataagg cggaagcagc 2220
caataagaag gagaaggcga atggctgaga tgaaaaacct gaaaattgag gtggtgcgct 2280
ataacccgaa agtcgatacc gcaccgcata gcgcattcta tgaagtgcct tatgacgcaa 2340
ctacctcatt actggatgcg ctgggctaca tcaaagacaa cctggcaccg gacctgagct 2400
accgctggtc ctgccgtatg gcgatttgtg gttcctgcgg catgatggtt aacaacgtgc 2460
caaaactggc atgtaaaacc ttcctgcgtg attacaccga cggtatgaag gttgaagcgt 2520
tagctaactt cccgattgaa cgcgatctgg tggtcgatat gacccacttc atcgaaagtc 2580
tggaagcgat caaaccgtac atcatcggca actcccgcac cgcggatcag ggtactaaca 2640
tccagacccc ggcgcagatg gcgaagtatc accagttctc cggttgcatc aactgtggtt 2700
tgtgctacgc cgcgtgcccg cagtttggcc tgaacccaga gttcatcggt ccggctgcca 2760
ttacgctggc gcatcgttat aacgaagata gccgcgacca cggtaagaag gagcgtatgg 2820
cgcagttgaa cagccagaac ggcgtatgga gctgtacttt cgtgggctac tgctccgaag 2880
tctgcccgaa acacgtcgat ccggctgcgg ccattcagca gggcaaagta gaaagttcga 2940
aagactttct tatcgcgacc ctgaaaccac gctaaggagt gcaacatgac gactaaacgt 3000
aaaccgtatg tacggccaat gacgtccacc tggtggaaaa aattgccgtt ttatcgcttt 3060

tacatgctgc gcgaaggcac ggcggttccg gctgtgtggt tcagcattga actgattttc 3120
gggctgtttg ccctgaaaaa tggcccggaa gcctgggcgg gattcgtcga ctttttacaa 3180
aacccggtta tcgtgatcat taacctgatc actctggcgg cagctctgct gcacaccaaa 3240
acctggtttg aactggcacc gaaagcggcc aatatcattg taaaagacga aaaaatggga 3300
ccagagccaa ttatcaaaag tctctgggcg gtaactgtgg ttgccaccat cgtaatcctg 3360
tttgttgccc tgtactggta aggagcctga gatgattaat ccaaatccaa agcgttctga 3420
cgaaccggta ttctggggcc tcttcggggc cggtggtatg tggagcgcca tcattgcgcc 3480
ggtgatgatc ctgctggtgg gtattctgct gccactgggg ttgtttccgg gtgatgcgct 3540
gagctacgag cgcgttctgg cgttcgcgca gagcttcatt ggtcgcgtat tcctgttcct 3600
gatgatcgtt ctgccgctgt ggtgtggttt acaccgtatg caccacgcga tgcacgatct 3660
gaaaatccac gtacctgcgg gcaaatgggt tttctacggt ctggctgcta tcctgacagt 3720
tgtcacgctg attggtgtcg ttacaatcta acgcatcgcc aatgtaaatc cggcccgcct 3780
atggcgggcc gttttgtatg gaaaccagac cctatgttca aaacgacgct ctgcgccttt 3840
taatacc 3847

<210> 19
<211> 16
<212> DNA
<213> Artificial

<220>
<223> primer

<400> 19
atatgaaacc cggtac 16

<210> 20
<211> 16
<212> DNA
<213> Artificial

<220>
<223> primer

<400> 20
cgggtttcat atgtac 16